(19) **Europäisches Patentamt / European Patent Office / Office européen des brevets**

(11) **EP 1 731 093 B1**

(12) **EUROPEAN PATENT SPECIFICATION**

(45) Date of publication and mention
of the grant of the patent:
**09.01.2013 Bulletin 2013/02**

(21) Application number: **05710016.6**

(22) Date of filing: **09.02.2005**

(51) Int Cl.:
*A61B 5/06* ⁽²⁰⁰⁶·⁰¹⁾    *A61B 5/07* ⁽²⁰⁰⁶·⁰¹⁾
*A61B 1/06* ⁽²⁰⁰⁶·⁰¹⁾    *A61B 1/05* ⁽²⁰⁰⁶·⁰¹⁾

(86) International application number:
**PCT/JP2005/001964**

(87) International publication number:
**WO 2005/092188 (06.10.2005 Gazette 2005/40)**

(54) **SYSTEM FOR DETECTING POSITION IN EXAMINEE**

SYSTEM ZUM NACHWEIS DER POSITION IN EINEM UNTERSUCHTEN

SYSTEME DE DETECTION D'UNE POSITION CHEZ UN CANDIDAT

(84) Designated Contracting States:
**DE FR GB**

(30) Priority: **29.03.2004 JP 2004095882**
**01.04.2004 JP 2004109049**

(43) Date of publication of application:
**13.12.2006 Bulletin 2006/50**

(73) Proprietor: **Olympus Corporation**
**Tokyo 151-0072 (JP)**

(72) Inventors:
  • **HONDA, Takemitsu**
    **Hino-shi**
    **Tokyo 191-0062 (JP)**
  • **HIRAKAWA, Katsumi**
    **Kanagawa 229-1103 (JP)**
  • **KIMOTO, Seiichiro**
    **Tokyo 192-0045 (JP)**
  • **NAGASE, Ayako**
    **Tokyo 192-0045 (JP)**

  • **SASAGAWA, Katsuyoshi**
    **Tokyo 191-0031 (JP)**
  • **SUZUKI, Katsuya**
    **Kanagawa 229-1103 (JP)**
  • **NAKATSUCHI, Kazutaka**
    **Tokyo 191-0062 (JP)**

(74) Representative: **von Hellfeld, Axel**
**Wuesthoff & Wuesthoff**
**Patent- und Rechtsanwälte**
**Schweigerstrasse 2**
**81541 München (DE)**

(56) References cited:
**WO-A1-97/29685      WO-A1-97/32179**
**WO-A1-99/49783      FR-A1- 2 842 721**
**JP-A- 5 200 015      JP-A- 2001 046 358**
**JP-A- 2004 041 709      JP-A- 2004 329 749**

Remarks:
The file contains technical information submitted after the application was filed and not included in this specification

EP 1 731 093 B1

**Description**

TECHNICAL FIELD

The present invention relates to an intra-subject position detection system which includes a subject insertable device which is introduced into a subject and moves through the subject, and a position detecting device which is arranged outside the subject and obtains position information of the subject insertable device in the subject.

BACKGROUND ART

In recent years, a swallowable-type capsule endoscope is proposed in the field of endoscope. The capsule endoscope is equipped with an imaging function and a radio communication function. The capsule endoscope is swallowed by the subject from a mouth for an observation (examination). After being swallowed, the capsule endoscope moves through inside body cavities, for example, internal organs such as a stomach and a small intestine, with peristaltic motion inside the subject, and sequentially picks up images inside the body cavities until being naturally discharged.

While moving through the body cavities, the capsule endoscope sequentially transmits data of the picked-up images of the inside to the outside by radio communication. The transmitted data is stored in an external memory. After swallowing the capsule endoscope, the subject carries a receiver which has radio communication function and memory function until the capsule endoscope is discharged, and can move freely.

After the capsule endoscope is discharged, a doctor or a nurse can retrieve the image data stored in the memory and watch images of the internal organs on a display monitor to make diagnosis.

Some of the above-described types of the capsule endoscopes are employed in combination with an external receiver which has a function of detecting a position of the capsule endoscope in the subject, so that a specific endoscopic image can be obtained, e.g., so that images of a specific internal organ inside the subject can be obtained. One known example of such a capsule endoscope system which is equipped with a position detection function utilizes an embedded radio communication function of the capsule endoscope. Specifically, a receiver having separate antenna elements is arranged outside the subject and the plural antenna elements receive a radio signal transmitted from the capsule endoscope. The capsule endoscope system detects the position of the capsule endoscope in the subject based on difference in strength of the received radio signal at respective antenna elements (see, for example, Patent Document 1).

Patent Document 1: JP-A No. 2003-19111 (KOKAI)

Document JP2004-41709 A discloses a capsule medical apparatus having a specific space setting unit which designates a specific space in vivo and a capsule which is inserted or swallowed in vivo. Further, the capsule medical apparatus has a recognizing unit which recognizes whether or not the capsule exists in the specific space set by the specific space setting unit and a control unit which controls a state of the capsule based on an output from the recognizing unit.

DISCLOSURE OF INVENTION

PROBLEM TO BE SOLVED BY THE INVENTION

The conventional capsule endoscope system, however, is disadvantageous in that the position of the capsule endoscope in the subject cannot be detected with high accuracy. Disadvantages of the conventional system will be described below in detail.

The conventional capsule endoscope system detects the position of the capsule endoscope in the subject based on distribution of received signal strength over the plural antenna elements provided in the receiver as described above. Such a position detection mechanism presupposes that the strength of the radio signal transmitted from the capsule endoscope is attenuated uniformly as a function of distance from the capsule endoscope, as described in the paragraph [0018] of Patent Document 1.

However, since the organ and the like existing between the capsule endoscope and the antenna elements have different relative permittivities and conductivities in reality, the attenuation rate of the radio signal strength largely varies depending on the type of the organ, for example. For example, when a liver, blood vessels, or the like exist between the capsule endoscope and the antenna elements, they absorb a large amount of radio signals. Then, the attenuation rate of the strength of the radio signal is increased compared to the attenuation rate at the time when the organ does not exist, and the accurate position detection is hindered.

The present invention is achieved in view of the foregoing, and an object of the present invention is to provide an intra-subject position detection system which can accurately detect the position of the subject insertable device, such as the capsule endoscope, which is placed inside the subject, regardless of the existence of the organs or the like.

MEANS FOR SOLVING PROBLEM

To solve the problems as described above and to achieve an object, an intra-subject position detection system as recited in claim 1 is provided.
The system includes a subject insertable device which is introduced into a subject and moves through the subject, and a position detecting device which is arranged outside the subject and obtains position information of the subject insertable device inside the subject. The subject insertable device includes a magnetic field generator that generates a magnetostatic field, and the position detecting device includes a magnetic field detector that is arranged on the subject at a time of use and detects a strength of the magnetostatic field output from the magnetic field generator, a reference sensor that derives a position of the magnetic field detector relative to a reference position on the subject, and a position deriving unit that derives a position of the subject insertable device inside the subject based on the magnetic field strength detected by the magnetic field detector and the position of the magnetic field detector detected by the reference sensor.

According to the present invention as recited in claim 1, the reference sensor which derives the position of the magnetic field detector, and the position deriving unit that derives the position of the subject insertable device based on the position of the magnetic field detector derived by the reference sensor and the like are provided. Therefore, even when the position of the magnetic field detector changes according to the changes in the position of the subject or the like, the position of the subject insertable device can be detected with accuracy.

Further, in the intra-subject position detection system, the reference sensor may derive a distance between the reference position and the position of the magnetic field detector, and derive a position of the magnetic field detector relative to the reference position based on the distance derived.

Further, in the intra-subject position detection system, the position detecting device may include a first radio unit whose positional relation with the magnetic field detector is fixed, and the reference sensor further include a second radio unit that transmits/receives a radio signal to/from the first radio unit, and a distance deriving unit that derives a distance between the reference position and the magnetic field detector based on a received signal strength of the radio signal at one of the first and the second radio units.

Further, in the intra-subject position detection system, the magnetic field detector may include a plurality of magnetic field detectors, the first radio unit may include a plurality of first radio units, and the reference sensor may further include a position information database that stores a correspondence between respective distances between the plural magnetic field detectors and the reference position and the position of the magnetic field detector on the subject, and a data extracting unit that extracts information on a position associated with the distance derived by the distance deriving unit from information stored in the position information database.

In the intra-subject position detection system , there may be a plurality of reference positions set thereon, the reference sensor may derive a distance between each of the plural reference positions and the magnetic field detector, and derive the position of the magnetic field detector based on the distances derived relative to the plural reference positions.

In the intra-subject position detection system, the reference sensor may further include an orientation determining unit that determines an orientation in which a received signal strength of the radio signal is highest when the radio signal is sent from the first radio unit, and the reference sensor may derive the position of the magnetic field detector based on the distance derived by the distance deriving unit and the orientation determined by the orientation determining unit.

In the intra-subject position detection system, the magnetic field detector may include a plurality of magnetic field detectors, the first radio unit may include a plurality of first radio units corresponding to the magnetic field detectors, and the second radio unit may transmit the radio signal to each of the plural first radio units in a time-multiplexed manner.

In the intra-subject position detection system, the magnetic field detector may include a plurality of magnetic field detectors, the first radio unit may include a plurality of first radio units corresponding to the magnetic field detectors, and the second radio unit may transmit the radio signal of a different frequency to each of the plural first radio units.

In the intra-subject position detection system, the subject insertable device my further include a predetermined intra-subject information obtaining unit that obtains intra-subject information, and a radio transmission unit that transmits the intra-subject information obtained by the intra-subject information obtaining unit by radio, and the position detecting device may further include a receiving unit that receives a radio signal containing the intra-subject information, the radio signal being sent from the radio transmission unit.

In the intra-subject position detection system, the intra-subject information obtaining unit may include an illuminating unit that illuminates an inside of the subject, and an imaging unit that obtains an image of an inside of the subject, the inside being illuminated by the illuminating unit.

In the intra-subject position detection system, the position detecting device may further include a storing unit that stores the image obtained by the imaging unit and a position of the subject insertable device at a time the image is obtained in association with each other.

An intra-subject position detection system may include a subject insertable device which is introduced into a subject and moves through the subject, and a position detecting device which is arranged outside the subject and obtains position information of the subject insertable device inside the subject. The subject insertable device may include a magnetostatic field generator that generates a magnetostatic field, and the position detecting device may include a magnetic field detector that detects a magnetic field strength, an alternating-current magnetic field generator that is fixed to a predetermined position relative to the subject, and outputs an alternating-current magnetic field which is used for derivation of a position of the magnetic field detector, a coordinate deriving unit that derives a position coordinate of the magnetic field detector based on an alternating-current magnetic field component of the magnetic field detected by the magnetic field detector, a distance deriving unit that derives a distance between the magnetic field detector and the subject insertable device based on a direct-current magnetic field component of the magnetic field detected by the magnetic field detector, and a position information deriving unit that derives the position of the subject insertable device inside the subject based on a result of derivation by the coordinate deriving unit and a result of derivation by the distance deriving unit.

There may be provided the alternating-current magnetic filed generator that outputs the alternating-current magnetic field used for the derivation of the position of the magnetic field detector, the coordinate deriving unit that derives the position coordinate of the magnetic field detector based on the alternating-current magnetic field component of the magnetic field detected by the magnetic field detector, and the distance deriving unit that derives the distance between the magnetic field detector and the subject insertable device based on the direct-current magnetic field component of the detected magnetic field. Therefore, even when the position of the magnetic field detector is shifted due to the changes in the position of the subject or the like, the position of the subject insertable device can be detected with accuracy.

Further, the intra-subject position detection system may further include an alternating-current magnetic field extracting unit that extracts the alternating-current magnetic field component from the magnetic field which is detected by the magnetic field detector to output the extracted alternating-current magnetic field component to the coordinate deriving unit, and a direct-current magnetic field extracting unit that extracts the direct-current magnetic field component from the magnetic field which is detected by the magnetic field detector to output the extracted direct-current magnetic field component to the distance deriving unit.

In the intra-subject position detection system, the coordinate deriving unit may derive the position coordinate of the magnetic field detector based on a difference value between the alternating-current magnetic field component detected by the magnetic field detector and a reference alternating-current signal which corresponds to the alternating-current magnetic field output from the alternating-current magnetic field generator.

In the intra-subject position detection system, the magnetostatic field generator may be arranged so that a travel direction of a line of magnetic force is fixed with respect to the subject insertable device, the position detecting device may further include a magnetic field direction detecting unit that detects a travel direction of the magnetostatic field generated by the magnetostatic field generator and an orientation detecting unit that detects an orientation of the subject insertable device inside the subject based on a magnetic field direction detected by the magnetic field detector.

The magnetic field direction detecting unit that detects the direction of the magnetostatic field generated from the magnetostatic field generator may be provided, and the orientation of the subject insertable device may be detected based on the detected magnetic field direction. Since the direction of the magnetostatic field which output from the magnetostatic field generator has correspondence with the orientation of the magnetostatic field generator, the detection of the direction of the magnetostatic field allows an accurate detection of the orientation of the subject insertable device which includes the magnetostatic field generator.

Further, in the intra-subject position detection system, the position detecting device may further include an orientation database that stores relation among a distance from a magnetostatic field generator, the travel direction of the line of magnetic force, and the orientation of the subject insertable device, and the orientation detecting unit detects the orientation of the subject insertable device using the orientation database.

Further, in the intra-subject position detection system, the subject insertable device may further include a predetermined intra-subject information obtaining unit that obtains the intra-subject information, and a radio transmission unit that transmits the intra-subject information which is obtained by the intra-subject information obtaining unit by radio, and the position detecting device may further include a reception unit that receives a radio signal which contains the intra-subject information transmitted from the radio transmission unit.

In the intra-subject position detection system, the reception unit may include a plurality of reception units, and the position detecting device may further include a selecting unit that selects a reception unit to be used for the reception of the radio signal based on the position and orientation of the subject insertable device, the position being derived by the position information deriving unit.

In the intra-subject position detection system the intra-subject information obtaining unit may include an illuminating unit that illuminates an inside of the subject, and an imaging unit that obtains an image of the inside of the subject, the inside being illuminated by the illuminating unit.

In the intra-subject position detection system, the position detecting device may further include a storing unit that stores the image obtained by the imaging unit and the position of the subject insertable device at a time the image is obtained in association with each other.

EFFECT OF THE INVENTION

The intra-subject position detection system according to the present invention includes a reference sensor which derives the position of the magnetic field detector and the position deriving unit that derives the position of the subject insertable device based on the position of the magnetic field detector derived by the reference sensor, and thus has an advantage that detects the accurate position of the subject insertable device even when the position of the magnetic field detector changes due to, for example, changes in the position/posture of the subject.

Further, the intra-subject position detection system according to the present invention includes the magnetic field direction detecting unit that detects the direction of the magnetostatic field generated from the magnetostatic field generator, and detects the orientation of the subject insertable device based on the detected magnetic field direction. The intra-subject position detection system has an advantage that detects accurate detection of the orientation of the subject insertable device which includes the magnetostatic field generator by detecting the direction of the magnetostatic field because the direction of the magnetostatic field which output from the magnetostatic field generator has correspondence with the orientation of the magnetostatic field generator.

BRIEF DESCRIPTION OF DRAWINGS

FIG. 1 is a schematic diagram of an overall structure of an intra-subject position detection system according to a first embodiment of the present invention;

FIG. 2 is a block diagram of a structure of a test capsule included in the intra-subject position detection system;

FIG. 3 is a block diagram of a structure of a magnetic field detector and a reference sensor both included in the intra-subject position detection system;

FIG. 4 is a block diagram of a structure of a position information deriving unit included in the intra-subject position detection system;

FIG. 5 is a flowchart of a position deriving operation of the magnetic field detector;

FIG. 6 is a flowchart showing how the position of the test capsule is derived;

FIG. 7 is a schematic diagram showing how the position of the test capsule is derived;

FIG. 8 is a schematic diagram of a structure of a magnetic field detector and a reference sensor both included in an intra-subject position detection system according to a second embodiment;

FIG. 9 is a block diagram of a structure of a reference sensor included in an intra-subject position detection system according to a third embodiment;

FIG. 10 is a block diagram of a structure of a reference sensor included in an intra-subject position detection system according to a fourth embodiment;

FIG. 11 is a block diagram of a structure of a capsule endoscope included in an intra-subject position detection system according to a fifth embodiment;

FIG. 12 is a block diagram of a structure of a position information deriving unit included in the intra-subject position detection system according to the fifth embodiment;

FIG. 13 is a schematic diagram of an overall structure of an intra-subject position detection system according to a first example;

FIG. 14 is a schematic diagram of a structure of a position information deriving unit included in the intra-subject position detection system according to the first example;

FIG. 15 is a flowchart of an operation of the position information deriving unit;

FIG. 16 is a schematic diagram showing how the position information deriving unit derives the position of the test capsule;

FIG. 17 is a schematic diagram of an overall structure of an intra-subject position detection system according to a second example;

FIG. 18 is a schematic diagram of a structure of a capsule endoscope included in the intra-subject position detection system according to the second example;

FIG. 19 is a schematic diagram of a structure of a position information deriving unit included in the intra-subject position detection system according to the second example;

FIG. 20 is a flowchart of an operation of the position information deriving unit; and

FIG. 21 is a schematic diagram showing how the position information deriving unit derives a direction toward which the capsule endoscope is oriented.

EXPLANATIONS OF LETTERS OR NUMERALS

| | |
|---|---|
| 1 | Subject |
| 2 | Test capsule |
| 3 | Position detecting device |
| 4 | Display device |
| 5 | Portable recording medium |
| 6a to 6h | Magnetic field detectors |
| 7 | Reference sensor |
| 9a, 9b | Fixing member |
| 10 | Position information deriving unit |
| 11 | Casing |
| 12 | Permanent magnet |
| 13 | Filling member |
| 15 | Magnetic field sensor |
| 16 | Radio transmission unit |
| 17 | Transmitter |
| 18 | Transmitting antenna |
| 19 | Radio reception unit |
| 20 | Control unit |
| 21 | Distance storage unit |
| 22 | Correspondence database |
| 23 | Output unit |
| 24 | Receiving antenna |
| 25 | Receiving circuit |
| 26 | Received signal strength detecting unit |
| 27 | Distance deriving unit |
| 28 | Position deriving unit |
| 30 | Strength comparator |
| 31 | Selector |
| 32 | Distance deriving unit |
| 33 | Position information storing unit |
| 34 | Capsule position calculator |
| 35 | Storage unit |
| 36a to 36h | Magnetic field detectors |
| 37 | Reference sensor |
| 38 | Control unit |
| 39 | Spectrum analyzer |
| 41 | Reference sensor |
| 42 to 44 | Receiving antennae |
| 45 | Selector |
| 46 | Control unit |
| 47 | Position deriving unit |
| 50 | Reference sensor |
| 51 | Array antenna |
| 52 | Radio reception unit |
| 53 | Orientation adjuster |
| 54 | Control unit |
| 55 | Capsule endoscope |
| 56 | LED |
| 57 | LED driving circuit |
| 58 | CCD |
| 59 | CCD driving circuit |
| 60 | Function executing unit |
| 61 | Transmitting circuit |
| 62 | Transmitting antenna unit |

| 63 | System control circuit |
|---|---|
| 64 | Receiving antenna unit |
| 65 | Separating circuit |
| 66 | Power regenerating circuit |
| 67 | Booster circuit |
| 68 | Capacitor |
| 69 | Control information detecting circuit |
| 70 | Position information deriving unit |
| 72 | Receiving circuit |
| 73 | Signal processing circuit |
| 74 | Storage unit |
| 75 | Oscillator |
| 76 | Control information input unit |
| 77 | Superposing circuit |
| 78 | Amplifier |
| 79 | Power supply unit |
| 108 | Position information deriving unit |
| 109 | Alternating-current magnetic field generator |
| 113 | LPF |
| 114 | Strength comparator |
| 115 | Selector |
| 116 | Distance deriving unit |
| 117 | DC component removing unit |
| 118 | Subtracter |
| 119 | Device coordinate deriving unit |
| 120 | Position calculator |
| 121 | Storage unit |
| 122 | Capsule endoscope |
| 123 | Position detecting device |
| 124a to 124h | Magnetic field detectors |
| 125 | Position information deriving unit |
| 126 | LED |
| 127 | LED driving circuit |
| 128 | CCD |
| 129 | CCD driving circuit |
| 130 | Transmitting circuit |
| 131 | Transmitting antenna unit |
| 132 | System control circuit |
| 133 | Receiving antenna unit |
| 134 | Separating circuit |
| 135 | Power regenerating circuit |
| 136 | Booster circuit |
| 137 | Capacitor |
| 138 | Control information detecting circuit |
| 139 | Function executing unit |
| 140 | Strength comparator |
| 141 | Selector |
| 142 | Distance deriving unit |
| 143 | Position calculator |
| 144 | Orientation database |
| 145 | Orientation detecting unit |
| 146 | DC component removing unit |
| 147 | Subtracter |
| 148 | Device coordinate deriving unit |
| 149 | Antenna selector |
| 150 | Receiving circuit |
| 151 | Signal processing unit |
| 152 | Storage unit |

| 153 | Oscillator |
| 154 | Control information input unit |
| 155 | Superposing circuit |
| 156 | Amplifier |
| 157 | Power supply unit |
| 158 | LPF |

BEST MODE(S) FOR CARRYING OUT THE INVENTION

Exemplary embodiments (hereinafter simply referred to as "embodiments") of an intra-subject position detection system according to the present invention will be described in detail below. It should be noted that the drawings are merely schematic and a ratio of width to thickness of each element and thickness ratio among different elements may be different in practice. Different drawings may show each of the elements in different dimension and in different reduction scale.

FIRST EMBODIMENT

An intra-subject position detection system according to a first embodiment will be described. The intra-subject position detection system according to the first embodiment includes a test capsule 2, a position detecting device 3, a display device 4, and a portable recording medium 5. The test capsule 2 is introduced into a subject 1 and functions as an example of a subject insertable device. The position detecting device 3 detects a position of the test capsule 2 in the subject 1. The display device 4 displays position information of the test capsule 2 as is detected by the position detecting device 3. The portable recording medium 5 serves to deliver information between the position detecting device 3 and the display device 4.

The display device 4 displays the position information of the test capsule 2 as is obtained by the position detecting device 3. The display device 4 has a structure like a workstation or the like to perform an image display based on the data delivered through the portable recording medium 5. More specifically, the display device 4 may directly display the image by CRT display, liquid crystal display, or the like; alternatively, the display device 4 may output the image to other media, such as a printer.

The portable recording medium 5 has such a structure that the portable recording medium 5 can be inserted into and removed out from the position information deriving unit 10 and the display device 4 described later, and information output and information recording can be carried out while the portable recording medium 5 is placed in one of the position information deriving unit 10 and the display device 4. Specifically, the portable recording medium 5 is inserted into the position information deriving unit 10 to record the information concerning the position of the test capsule 2 while the test capsule 2 is moving through the body cavity of the subject 1. After the test capsule 2 is discharged from the subject 1, the portable recording medium 5 is removed out from the position information deriving unit 10 and inserted into the display device 4. Then, the display device 4 reads out the recorded data from the portable recording medium 5. When the data delivery between the position information deriving unit 10 and the display device 4 is realized by the portable recording medium 5 such as a Compact Flash (registered trademark) memory, the subject 1 can move freely even while the test capsule 2 is moving through inside the subject 1, dissimilar to a system where data delivery is carried out by a cable connection between the position information deriving unit 10 and the display device 4.

Before a capsule endoscope or the like is introduced into the subject 1, a preliminary examination is carried out with the test capsule 2. The test capsule 2 checks whether there is a portion with stenosis, where passage of the capsule endoscope is difficult, in the subject or not. The intra-subject position detection system according to the first embodiment examines how the test capsule 2 moves through inside the subject 1, and has a highly accurate position detection mechanism to realize such examination.

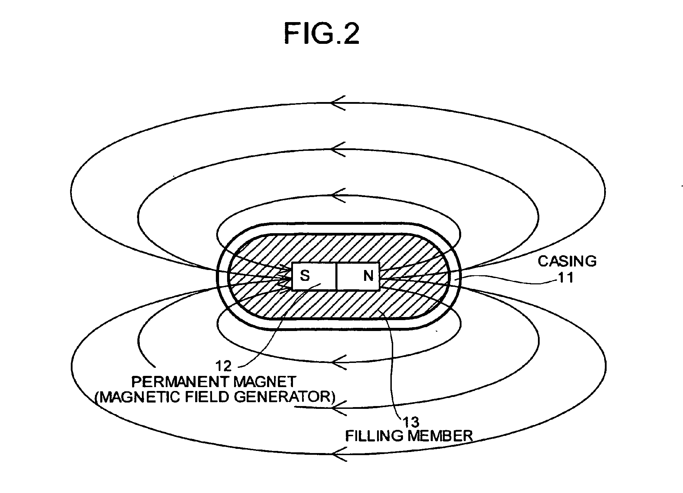

FIG. 2 is a schematic diagram of a structure of the test capsule 2. As shown in FIG. 2, the test capsule 2 includes a casing 11, a permanent magnet 12, and a filling member 13. The casing 11 is formed in a capsule shape similar to a shape of a casing of the capsule endoscope. The permanent magnet is arranged inside the casing 11. The filling member 13 serves to fill up a gap between an inner surface of the casing 11 and the permanent magnet 12.

The casing 11 is formed, for example, of a biocompatible material so that the living subject 1 would not suffer from a harmful influence even when the test capsule 2 stays inside the subject for a few days.

The permanent magnet 12 functions as a magnetic field generator recited in the appended claims. The permanent magnet 12 is formed of a permanent magnet of a size accommodatable inside the casing 11. The permanent magnet 12 serves to generate a magnetostatic field with a magnetic strength whose temporal variation is ignorable. When the test capsule provided with the magnetic field generator moves, a magnetic field changes therewith. In the first embodiment, however, since the position of the magnetic field generator does not change much during a time period of magnetic strength detection, the magnetic field generator generates a constant magnetic field. Instead of employing the permanent magnet 12, it may be possible to employ a coil or the like as the magnetic field generator. The coil, for example, forms a magnetostatic field in response to the supply of constant electric currents. It is preferable though to form the magnetic field generator with the permanent magnet 12 since the permanent magnet 12 is advantageous, for example, in that it does not require driving electricity.

The magnetostatic field generated from the permanent magnet 12 can be represented by closed-loop-like lines of magnetic force. The line of magnetic force flows from a north (N) pole side, passes through the outside of the permanent magnet 12, and comes back to a south (S) pole side as shown in FIG. 2. A traveling direction of the line of magnetic force has a locus-dependency as can be seen from FIG. 2. It is possible to assume, however, that the strength of the magnetostatic field represented by the density of lines of magnetic force is determined as a function of distance from the test capsule 2 alone. Specifically, since the permanent magnet 12 housed inside the test capsule 2 is extremely small to an ignorable extent in comparison with the distance between the test capsule 2 and the magnetic field detectors 6a to 6h, magnetic field strength P at a position which is distance r away from the test capsule 2 can be represented as:

$$P = \alpha/r^3 \qquad\qquad\qquad (1)$$

where $\alpha$ is proportionality coefficient.

The intra-subject position detection system according to the first embodiment detects the position of the test capsule 2 based on the relation represented by the above equation (1) as described later.

The filling member 13 serves to fill up the space between the inner surface of the casing 11 and the permanent magnet 12, to secure the permanent magnet 12 at a predetermined position. The filling member 13 is formed of a material which does not have a negative influence on the subject 1. For example, the filling member 13 is formed of a barium sulfate. Since the barium sulfate is also usable as an X-ray contrast agent, the position of the test capsule 2 can be detected also by the X-ray in addition to the position detecting manner of the first embodiment. When results of position detection of the first embodiment and of the X-ray are compared with each other, more accurate position detection can be realized. The use of barium sulfate as the filling member 13 of the first embodiment is not essential. Needless to say, any material is usable as far as the material serves as a filling member.

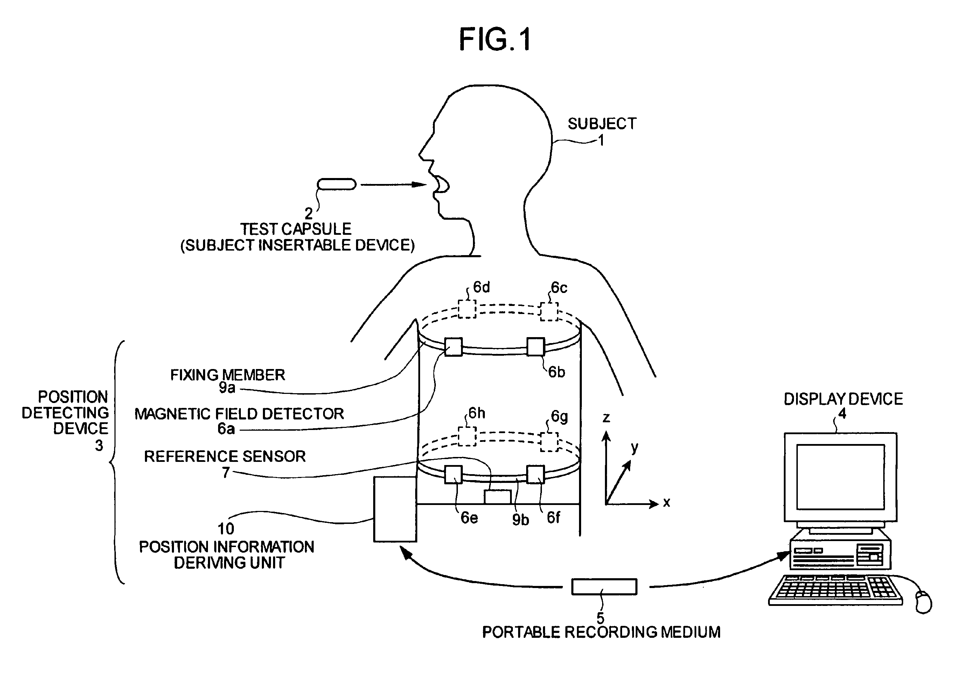

The position detecting device 3 will be described. The position detecting device 3 includes, as shown in FIG. 1, magnetic field detectors 6a to 6h (hereinafter, sometimes collectively referred to as "magnetic field detector 6"), a reference sensor 7, fixing members 9a and 9b, and a position information deriving unit 10. The magnetic field detectors 6a to 6h serve to detect magnetostatic field generated from the permanent magnet 12 housed in the test capsule 2. The reference sensor 7 serves to detect positions of the magnetic field detectors 6a to 6h. The fixing members 9a and 9b serve to hold the magnetic field detectors 6a to 6h on a surface of the subject 1. The position information deriving unit 10 derives the position of the test capsule 2 inside the subject 1.

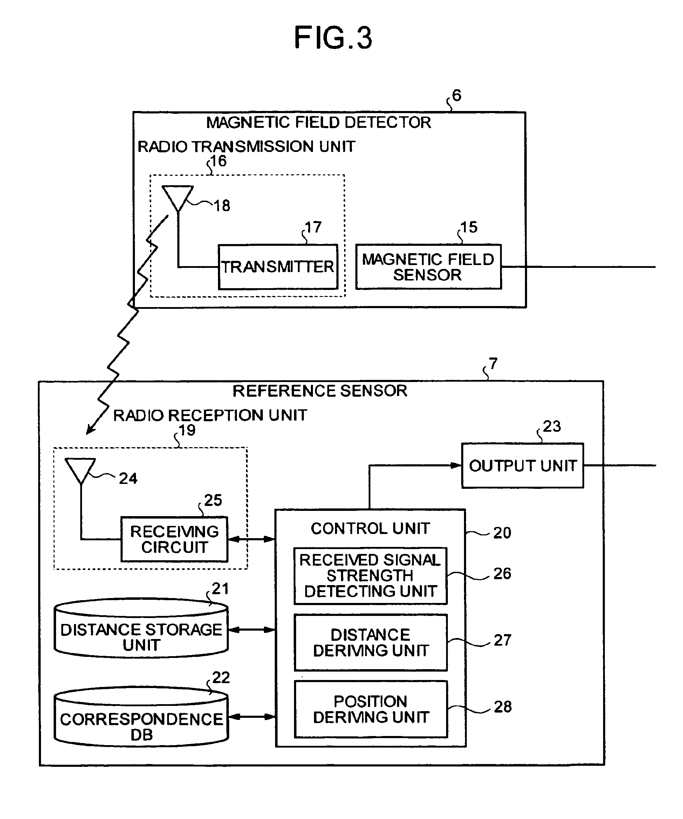

FIG. 3 is a block diagram showing a detailed structure of the magnetic field detector 6 and the reference sensor 7. The magnetic field detector 6 includes a magnetic field sensor 15 and a radio transmission unit 16 which performs radio transmission with the reference sensor 7. In the first embodiment, the magnetic field sensor 15 and the radio transmission unit 16 are arranged adjacent with each other on a same base, for example. Even when the subject 1 changes position, the positional relation between the magnetic field sensor 15 and the radio transmission unit 16 is maintained.

The magnetic field sensor 15 serves to detect a magnetic field at a position where the magnetic field detector 6 is arranged. Specifically, the magnetic field detectors 6a to 6h include a Magneto Impedance (MI) sensor, for example. The MI sensor has a structure including a FeCoSiB based amorphous wire, for example, as a magneto-sensitive medium. When a high-frequency electric current is applied to the magneto-sensitive medium, a magnetic impedance of the magneto-sensitive medium dramatically changes due to an external magnetic field. This phenomenon is called MI effect. The MI sensor utilizes the MI effect to detect the magnetic field strength. Though other types of sensors may be employed for the magnetic field detectors 6a to 6h, the use of the MI sensor is advantageous in that a particularly highly sensitive detection of the magnetic field strength can be realized.

The radio transmission unit 16 serves to send electric waves to the reference sensor 7 for the detection of the position of the magnetic field detector 6. Specifically, the radio transmission unit 16 includes a transmitter 17 and a transmitting antenna 18. The transmitter 17 generates a radio signal to be transmitted. The transmitting antenna 18 transmits the radio signal generated by the transmitter 17. The radio transmission unit 16 has a function of transmitting the radio signal of a predetermined strength to the reference sensor 7. In the first embodiment, plural magnetic field detectors are arranged as the magnetic field detector 6. The radio transmission unit 16 arranged in each of the magnetic field detectors 6a to 6h sends the radio signal to the reference sensor 7 in a time-multiplexed manner. In other words, in the first embodiment, the radio transmission units 16 in the respective magnetic field detectors 6a to 6h sequentially send the radio signals in a predetermined order in order to avoid simultaneous transmission of the radio signals from the plural magnetic field detectors 6.

The reference sensor 7 will be described. The reference sensor 7 serves to detect the position of each of the magnetic field detectors 6a to 6h. Specifically, the reference sensor 7 includes a radio reception unit 19, a control unit 20, a distance storage unit 21, a correspondence database 22, and an output unit 23. The radio reception unit 19 serves as a second radio unit and has a function of receiving a radio signal transmitted from each of the magnetic field detectors 6a to 6h. The control unit 20 serves to derive a distance from each of the magnetic field detectors 6a to 6h and a position of the magnetic field detectors 6a to 6h. The distance storage unit 21 stores the distance between the reference sensor 7 and each of the magnetic field detectors 6a to 6h, as derived by the control unit 20. The correspondence database 22 is employed by the control unit 20 to derive the positions of the magnetic field detectors 6a to 6h. The output unit 23 outputs the positions of the magnetic field detectors 6a to 6h as derived to the position information deriving unit 10.

The radio reception unit 19 receives a radio signal sent from the radio transmission unit 16 provided in each of the magnetic field detectors 6a to 6h and outputs the received signal to the control unit 20. Specifically, the radio reception unit 19 includes a receiving antenna 24 and a receiving circuit 25. At least the receiving antenna 24 is arranged at a reference point. The reference point remains at a predetermined distance away from the organs or the like of the subject 1 regardless of the position/posture of the subject 1.

The control unit 20, based on the strength of the radio signal received by the radio reception unit 19, derives the distance between the reference sensor 7 (more accurately, the receiving antenna 24) and the magnetic field detectors 6a to 6h (more accurately, the transmitting antenna 18). At the same time, the control unit 20 derives the positions of the magnetic field detectors 6a to 6h using the result of the distance derivation. Specifically, the control unit 20 includes a received signal strength detecting unit 26, a distance calculator 27, and a position deriving unit 28. The received signal strength detecting unit 26 detects the strength of the received radio signal. The distance deriving unit 27 derives the distance from each of the magnetic field detectors 6a to 6h based on the received signal strength obtained by the received signal strength detecting unit 26. The position deriving unit 28 derives the position of each of the magnetic field detectors 6a to 6h based on the information on the distance derived by the distance deriving unit 27 and information stored in the correspondence database 22.

The distance storage unit 21 serves to store the distance derived by the distance deriving unit 27. In the first embodiment, firstly the distance from each of the magnetic field detectors 6a to 6h is derived; and thereafter the position of each of the magnetic field detectors 6a to 6h is derived. While the distance is derived with respect to the magnetic field detector 6a to 6h, the already derived distance is held by the distance storage unit 21.

The correspondence database 22 serves to derive a specific position of each of the magnetic field detectors 6a to 6h based on the distance between the reference sensor 7 and each of the magnetic field detectors 6a to 6h. The correspondence database 22 may store any contents describing the correspondence between the distance and the position. In the first embodiment, however, focusing on the relation between the positional changes and the changes in distance between the reference sensor 7 and each of the magnetic field detectors 6a to 6h accompanying the changes in the position of the subject 1 or the like, the correspondence database 22 stores correspondence between all the distances between the magnetic field detectors 6a to 6h and the reference sensor 7, and all the positions of the magnetic field detectors 6a to 6h.

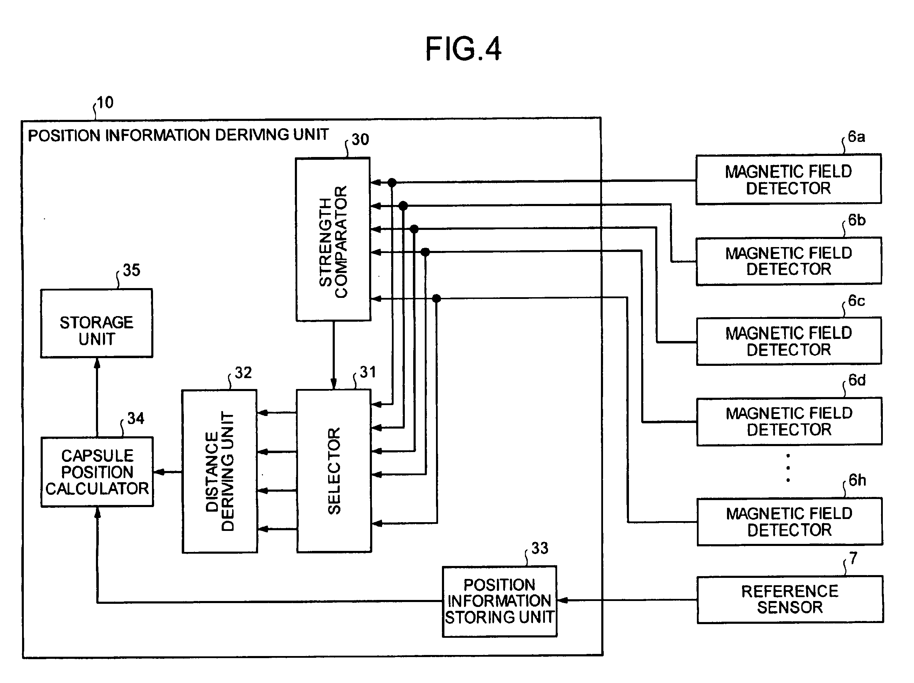

The position information deriving unit 10 will be described. FIG. 4 is a block diagram of a structure of the position information deriving unit 10. The position information deriving unit 10 includes, as shown in FIG. 4, a strength comparator 30, a selector 31, and a distance deriving unit 32. The strength comparator 30 compares strengths of the magnetic fields detected by the magnetic field detectors 6a to 6h. The selector 31 selects a part of the result of detection by the magnetic field detectors 6a to 6h and outputs the selected part based on the result of comparison by the strength comparator 30. The distance deriving unit 32 derives a distance between the test capsule 2 and a selected magnetic field detector 6 based on the strength of the magnetic field selected by the selector 31. The position information deriving unit 10 further includes a position information storing unit 33, a capsule position calculator 34, and a storage unit 35. The position information storing unit 33 holds information related to the positions of the magnetic field detectors 6a to 6h as supplied from the reference sensor 7. The capsule position calculator 34 derives the position of the test capsule 2 by a predetermined operation based on the distance between the magnetic field detectors 6a to 6h as derived by the distance deriving unit 32 and the position information of the magnetic field detectors 6a to 6h as stored in the position information storing unit 33. The storage unit 35 stores the result of operation.

The selector 31 selects a part of the plural magnetic field detectors 6a to 6h. The selector 31 outputs the strength of the magnetic field detected by the selected magnetic field detector 6 to the distance deriving unit 32. Any selection algorithm can be employed for the selector 31. In the first embodiment, however, the selector 31 selects three magnetic field detectors 6 that detect stronger magnetic fields, and outputs the strength of the magnetic fields detected by the selected magnetic field detectors 6.

The distance deriving unit 32 serves to derive a distance between the test capsule 2 and the magnetic field detector 6 selected by the selector 31 based on the value of magnetic field strength supplied via the selector 31. Specifically, the distance deriving unit 32 derives the distance between the test capsule 2 and the magnetic field detector 6 based on the supplied value of the magnetic field strength and the equation (1).

The capsule position calculator 34 serves to derive the position of the test capsule 2 by carrying out a predetermined calculation using the distance derived by the distance deriving unit 32 and the position information of the magnetic field detectors 6a to 6h stored in the position information storing unit 33. The capsule position calculator 34 further has a function of supplying the derived position of the test capsule 2 to the storage unit 35.

The storage unit 35 serves to store the derived position of the test capsule 2. Specifically, the storage unit 35 has a function of supplying the information input from the capsule position calculator 34 to the portable recording medium 5.

An operation of the intra-subject position detection system of the first embodiment will be described. The intra-subject position detection system of the first embodiment has functions of deriving the positions of the magnetic field detectors 6a to 6h using the reference sensor 7 and deriving the position of the test capsule 2 based on the detected positions of the magnetic field detectors 6a to 6h and the magnetic field strengths detected by the magnetic field detectors 6a to 6h. In the following, the derivation of the positions of the magnetic field detectors 6a to 6h by the reference sensor 7 and the derivation of the position of the test capsule 2 by the position information deriving unit 10 will be described sequentially.

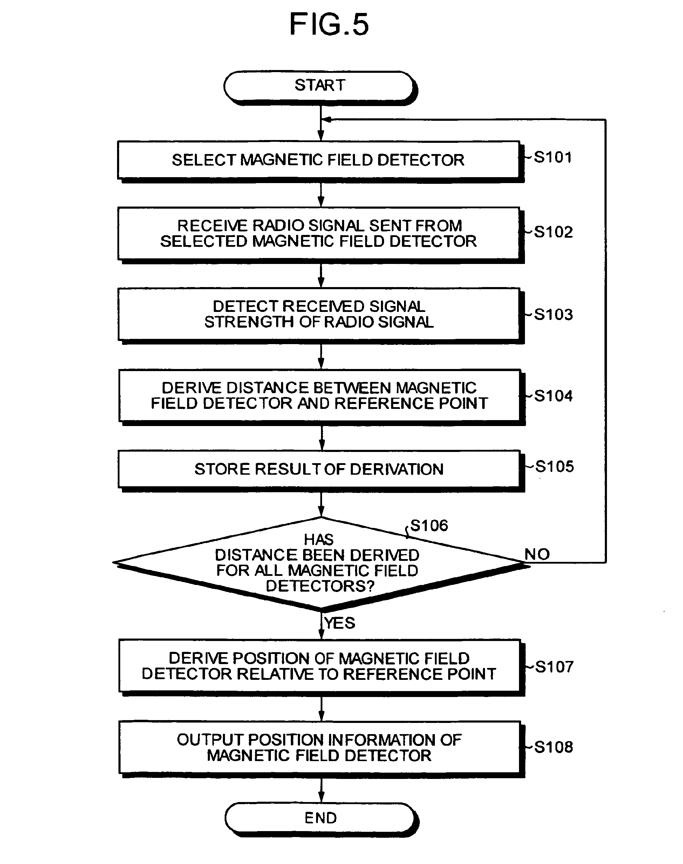

FIG. 5 is a flowchart of a derivation operation of the positions of the magnetic field detectors 6a to 6h by the reference sensor 7. As shown in FIG. 5, the reference sensor 7 first selects a predetermined magnetic field detector 6 (step S101), and receives the radio signal transmitted from the radio transmission unit 16 of the selected magnetic field detector 6 by the radio reception unit 19 (step S102). Then, the received signal strength detecting unit 26 detects the strength of the received radio signal (step S103). The distance deriving unit 27 derives the distance between the selected magnetic field detector 6 and the reference point based on the detected strength (step S104).

Thereafter, the reference sensor 7 stores the result of the derivation in the distance storage unit 21 (step S105), and determines whether the derivation of the distances between the reference point and all of the magnetic field detectors 6a to 6h have been completed or not (step S106). When the reference sensor 7 determines that the distance derivation has not been completed (No in step S106), the process returns to step S101. Then, the reference sensor 7 selects one of the magnetic field detectors 6 for which the distance derivation has not been performed, and repeats the process as described above. When the reference sensor 7 determines that the distance derivation has been completed (Yes in step S106), the reference sensor 7 derives the positions of the magnetic field detectors 6a to 6h relative to the reference point based on the distances between the reference point and the magnetic field detectors 6a to 6h and information stored in the correspondence database 22 (Step S107), and outputs information concerning the positions of the magnetic field detectors 6a to 6h to the position information deriving unit 10 through the output unit 23 (step S108).

The distance derivation in step S104 will be described in brief. The radio transmission unit 16 in each of the magnetic field detectors 6a to 6h has a function of radially transmitting the radio signal. The strength of the transmitted radio signal is proportional to the -$3^{rd}$ power of the traveled distance thereof. The distance deriving unit 27 derives the distance between the reference point and the magnetic field detector 6 based on the strength of the received radio signal detected by the received signal strength detecting unit 26 according to the relation as described above.

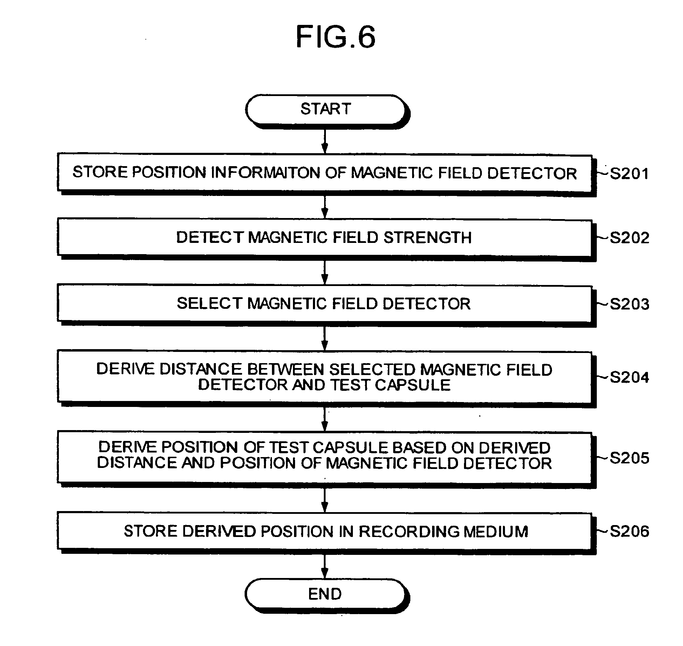

The derivation of the position of the test capsule 2 by the position information deriving unit 10 will be described. FIG. 6 is a flowchart of the derivation of the position of the test capsule 2 by the position information deriving unit 10. As shown in FIG. 6, the position information deriving unit 10 first stores the information concerning the positions of the magnetic field detectors 6a to 6h in the position information storing unit 33 (step S201). The information of the positions is derived by the reference sensor 7. The position information deriving unit 10 detects the strength of the magnetostatic field which is generated by the permanent magnet 12 in the test capsule 2 and detected by the magnetic field detectors 6a to 6h (step S202). The selector 31 selects the magnetic field detector 6 based on the detected strength (step S203).

Thereafter, the position information deriving unit 10 derives the distance between the selected magnetic field detector 6 and the test capsule 2 (Step S204), derives the position of the test capsule 2 based on the derived distance and the position of the selected magnetic field detector 6 (step S205), and stores the derived position of the test capsule 2 in the portable recording medium 5 through the storage unit 35 (step S206). The operation from step S201 to step S206 is repeated until the test capsule 2 is discharged outside the subject 1. The portable recording medium 5 stores information concerning the position of the test capsule 2 at each time.

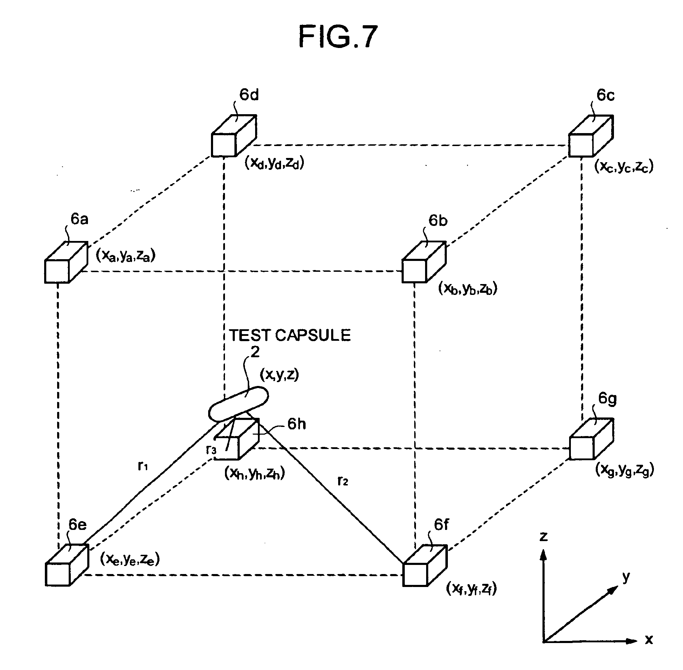

The derivation of the position of the test capsule 2 in step S205 will be briefly described. FIG. 7 is a schematic diagram for explaining the operation of derivation of the position of the test capsule 2. It is assumed that the positions of all the magnetic field detectors 6a to 6h are derived in step S107, and the respective positions are represented by coordinates $(x_a, y_a, z_a)$ to $(x_h, y_h, z_h)$ as shown in FIG. 7. Further, it is assumed that the magnetic field detectors 6e, 6f, and 6h are selected in step S203, and the distances between the test capsule 2 and the magnetic field detectors 6e, 6f, and 6h are found to be $r_1$, $r_2$, and $r_3$, respectively in step S204.

Under the above-described conditions, the position coordinate $(x, y, z)$ of the test capsule 2 can be derived based on the following equation:

$$(x - x_e)^2 + (y - y_e)^2 + (z - z_e)^2 = r_1^2 \qquad (2)$$

$$(x - x_f)^2 + (y - y_f)^2 + (z - z_f)^2 = r_2^2 \qquad (3)$$

$$(x - x_h)^2 + (y - y_h)^2 + (z - z_h)^2 = r_3^2 \qquad (4)$$

where $(x_e, y_e, z_e)$, $(x_f, y_f, z_f)$, $(x_h, y_h, z_h)$ represent coordinates of the magnetic field detectors 6e, 6f, and 6h, respectively, and $r_1$, $r_2$, and $r_3$ represent distances. Since specific values are assigned to $x_e$, $x_f$, $x_h$, $y_e$, $y_f$, $y_h$, $z_e$, $z_f$, $z_h$, and $r_1$, $r_2$, and $r_3$ of the equations (2) to (4) in step S107 and S204, only three variants remains unknown in the equations (2) to (4), i.e., x, y, z. When the equations (2) to (4) are regarded as simultaneous equations and solved, the position of the test capsule 2 can be found.

Advantages of the intra-subject position detection system of the first embodiment will be described. The intra-subject position detection system of the first embodiment includes the permanent magnet 12 in the test capsule 2, and detects the position of the test capsule 2 in the subject 1 based on the detected strength of the magnetostatic field generated by the permanent magnet 12. Different from the electromagnetic waves or the like, the magnetostatic field has a characteristic of constant attenuation regardless of the fluctuation in relative permittivity in propagating area. Hence, the relation represented by the equation (1) holds well. Therefore, even when the position detection is performed in a space where objects having different relative permittivities exist, e.g., inside the human body where internal organs having different relative permittivities exist, the position detection can be realized with a higher accuracy compared with the level of accuracy obtained by the position detection by electromagnetic waves or the like.

Another advantage of the position detection by the magnetostatic field is an alleviation of pain of the subject 1 at the time of introduction of the test capsule 2. Due to the above-described characteristics, the intra-subject position detection system of the first embodiment can suppress the deterioration of the accuracy in position detection caused by the changes in surrounding environment. Therefore, different from other examination system in which the subject needs to refrain from eating and drinking, the intra-subject position detection system of the first embodiment does not impose restriction on the subject 1 at a time of the introduction of the test capsule 2, for example. Thus, the subject 1 can lead a normal life even while the subject 1 is under the examination by the test capsule 2, whereby the pains of the subject 1 caused by the examination can be alleviated.

Further, the intra-subject position detection system of the first embodiment includes the reference sensor 7 which derives the positions of the magnetic field detectors 6a to 6h that detect the strength of the magnetostatic field generated by the test capsule 2. As described above, the magnetic field detectors 6a to 6h are arranged on the body surface of the subject 1. The positions of the magnetic field detectors 6a to 6h may be shifted with respect to the subject 1 over time and due to the changes in the position of the subject 1, for example. Therefore, the positions of the magnetic field detectors 6a to 6h are actually derived by the reference sensor 7, and the position of the test capsule 2 is derived based on the derived positions of the magnetic field detectors 6a to 6h, whereby the position of the test capsule 2 can be accurately detected regardless of the changes in the position of the subject 1, for example.

Further, the intra-subject position detection system of the first embodiment uses the radio signal for the derivation of the positions of the magnetic field detectors 6a to 6h, different from a system that uses the magnetostatic field for the derivation of the position of the test capsule 2. Since the radio signal and the magnetostatic field do not interfere with each other and are independently transmitted of each other, the intra-subject position detection system of the first embodiment can prevent the position derivation of the magnetic field detectors 6a to 6h from negatively affecting the position derivation of the test capsule 2. Therefore, the intra-subject position detection system of the first embodiment can perform the position derivation of the magnetic field detectors 6a to 6h without affecting the position derivation of the test capsule 2 even after the introduction of the test capsule 2 into the subject 1.

In practice, different from the position derivation of the test capsule 2, the position derivation of the magnetic field detectors 6a to 6h based on the radio signal is not substantially affected by the changes in attenuation of the radio signal caused by the objects inside subject 1. Different from the test capsule 2 which travels through a wide range from the esophagus to the large intestine, the magnetic field detectors 6a to 6h are not largely shifted even if the subject 1 moves. In addition, the objects between the reference sensor 7 and the magnetic field detectors 6a to 6h mostly remain the same regardless of the positional change. If the intra-subject position detection system has a function of comparing the strength of the radio signal transmitted from the magnetic field detectors 6a to 6h at an initial state and the strength at the time of position detection, an error in position derivation due to the difference in attenuation rate can be reduced.

SECOND EMBODIMENT

An intra-subject position detection system according to a second embodiment will be described. In the intra-subject position detection system according to the second embodiment, the radio signal transmitted from each of the magnetic field detectors 6a to 6h has a different frequency from each other, and the reference sensor simultaneously derives the distance between each of the magnetic field detectors 6a to 6h and the reference point depending on the difference in frequencies. In the second embodiment, the test capsule 2, the display device 4, the portable recording medium 5, the fixing member 9, and the position information deriving unit 10 have the same structures as those in the first embodiment, hence these elements are not shown in the drawings and the description thereof will not be repeated.

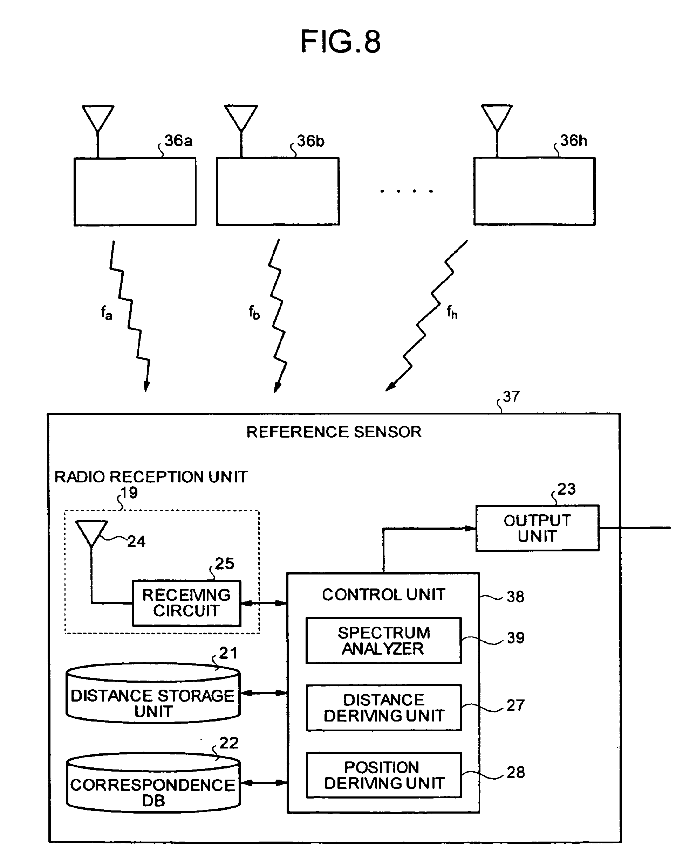

FIG. 8 is a block diagram showing the structures of the magnetic field detector and the reference sensor in the intra-subject position detection system according to the second embodiment. As shown in FIG. 8, magnetic field detectors 36a to 36h of the second embodiment have functions of transmitting radio signals of different frequencies $f_a$ to $f_h$, respectively, and of simultaneously transmitting such radio signals.

On the other hand, a reference sensor 37 of the second embodiment includes a spectrum analyzer 39 in place of the received signal strength detecting unit 26 in a control unit 38, in addition to the structure of the reference sensor 7 of the first embodiment. The spectrum analyzer 39 has functions of performing a frequency analysis based on the radio signal received by the radio reception unit 19, and of detecting received signal strength with respect to each of the frequency components $f_a$ to $f_h$. Thus, the control unit 38 detects the received signal strength with respect to the radio signals sent respectively from the magnetic field detectors 36a to 36h, and performs the distance derivation based on the received signal strength and the position derivation based on the distance and the correspondence similarly to the first embodiment.

Advantages of the intra-subject position detection system according to the second embodiment will be described. In the second embodiment, the magnetic field detectors 36a to 36h transmit the radio signals with different frequencies, and the reference sensor 37 detects the received signal strength for each frequency component using the spectrum analyzer 39. Having the above-described structure, the intra-subject position detection system of the second embodiment can detect the received signal strength of each of the radio signal by separating the radio signals transmitted from the magnetic field detectors 36a to 36h even when the magnetic field detectors 36a to 36h simultaneously send the radio signals. Therefore, the intra-subject position detection system of the second embodiment can employ the magnetic field detectors 36a to 36h that simultaneously transmit the radio signals, whereby the time required for the position derivation of the magnetic field detectors 36a to 36h can be reduced.

THIRD EMBODIMENT

An intra-subject position detection system according to a third embodiment will be described. In the intra-subject position detection system of the third embodiment, plural reference points are set. Preferably, three or more reference points are set. The reference sensor includes plural receiving antennae corresponding to the plural reference points, respectively. In the intra-subject position detection system of the third embodiment, elements other than the reference sensor are the same as those in the first and the second embodiments, and these elements are not shown in the drawings and the description thereof will not be repeated.

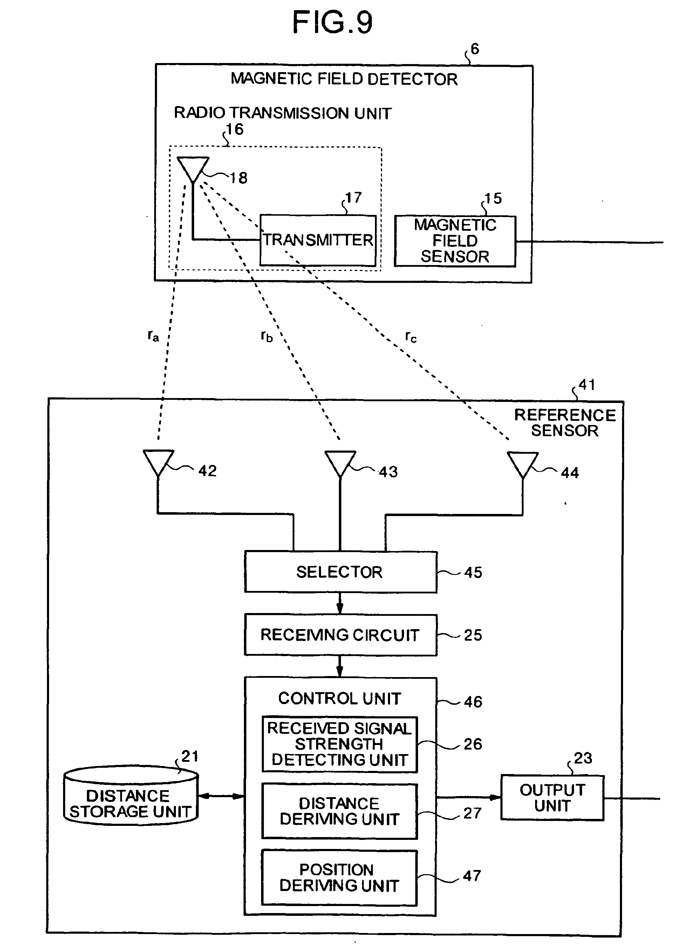

FIG. 9 is a block diagram showing a structure and a function of the intra-subject position detection system of the third embodiment. As shown in FIG. 9, a reference sensor 41 includes receiving antennae 42 to 44, a selector 45, and a control unit 46. The receiving antennae 42 to 44 are arranged corresponding to plural reference positions. The selector 45 is arranged between the receiving antennae 42 to 44 and the receiving circuit 25. The control unit 46 includes a position deriving unit 47 which performs position derivation based on a different algorithm from the algorithm employed by the position deriving unit 28 of the first and the second embodiments.

The operation of position derivation by the magnetic field detector 6 of the third embodiment will be briefly described. In the third embodiment, the magnetic field detector 6 sends a radio signal which is received through the receiving antennae 42 to 44. The selector 45 sequentially outputs the radio signals received through the receiving antennae 42 to 44 to the receiving circuit 25. The receiving circuit 25 extracts the strength of each radio signal and outputs the same to the control unit 46. The distance deriving unit 27 in the control unit 46 derives distances $r_a$, $r_b$, and $r_c$, between the respective set reference positions and the magnetic field detector 6. The values of the distances are stored in the distance storage unit 21.

The operation of the position deriving unit 47 will be described. The position deriving unit 47 grasps specific positions of the respective reference positions (more strictly, the positions of the receiving antennae 42 to 44) in advance. For example, the position deriving unit 47 grasps position coordinates. Based on the position coordinates of the receiving antennae 42 to 44 and the distances $r_a$, $r_b$, and $r_c$ between the respective receiving antennae 42 to 44 and the magnetic field detector 6, the position deriving unit 47 derives the position of the magnetic field detector 6. Specifically, when the position coordinates of the receiving antennae 42 to 44 are represented respectively as $(x_1, y_1, z_1)$, $(x_2, y_2, z_2)$, $(x_3, y_3, z_3)$

and the position coordinate of the magnetic detector 6 is represented as (x, y, z), following equations are satisfied:

$$(x - x_1)^2 + (y - y_1)^2 + (z - z_1)^2 = r_a^2 \qquad (5)$$

$$(x - x_2)^2 + (y - y_2)^2 + (z - z_2)^2 = r_b^2 \qquad (6)$$

$$(x - x_3)^2 + (y - y_3)^2 + (z - z_3)^2 = r_c^2 \qquad (7)$$

Three letters x, y, and z are unknown in the equations (5) to (7). The specific position of the magnetic field detector 6 can be derived by solving the equations (5) to (7).

By performing the position derivation of the magnetic field detector 6 in the above-described manner, the intra-subject position detection system of the third embodiment can realize the position derivation of the magnetic field detector 6 without using the correspondence database. Further, since the reference sensor 41 has the function of performing the position derivation based only on the radio signals received through the plural receiving antennae 42 to 44 without using previously categorically derived correspondence, the intra-subject position detection system of the third embodiment can realize even more accurate position derivation of the magnetic field detector 6 by accommodating individual difference of the movement of the subject 1. As a result, the intra-subject position detection system of the third embodiment can realize even more accurate position derivation of the test capsule 2.

FOURTH EMBODIMENT

An intra-subject position detection system according to a fourth embodiment will be described. In the intra-subject position detection system of the fourth embodiment, the reference sensor detects not only the strength of the radio signals sent from the magnetic field detector 6 but also a direction of a signal sender. In the intra-subject position detection system of the fourth embodiment, elements other than the reference sensor are the same as those in the first and second embodiments. Hence, these elements are not shown in the drawings and the description thereof will not be repeated.

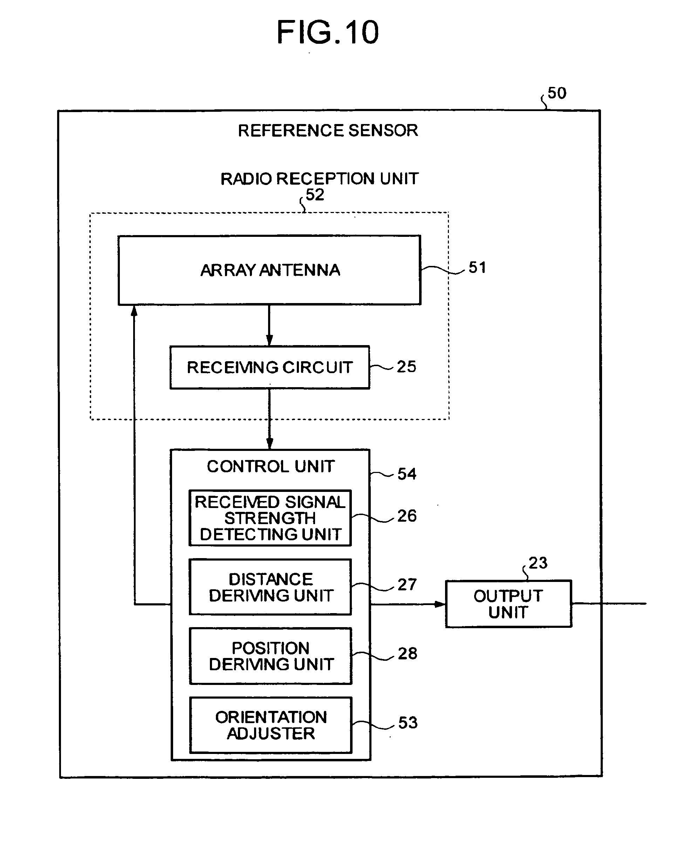

FIG. 10 is a block diagram of a structure of a reference sensor 50 included in the intra-subject position detection system of the fourth embodiment. As shown in FIG. 10, the reference sensor 50 includes a radio reception unit 52, a control unit 54, and the output unit 23. The radio reception unit 52 has an array antenna 51 which is employed instead of the receiving antenna 24 in the first embodiment. The control unit 54 has an orientation adjuster 53 as a new element.

The array antenna 51 serves to detect a direction of the magnetic field detector 6 which sends the radio signal on receiving the radio signal sent from the magnetic field detector 6. Specifically, the array antenna 51 includes plural receiving antennae, and a signal processing mechanism. The receiving antennae are arranged in a two-dimensional matrix, for example. The signal processing mechanism performs processing such as amplification and delaying on the radio signal received by the respective receiving antennae, to give the array antenna 51 as a whole a good receiver sensitivity in a predetermined direction (hereinafter referred to as "orientation"). The orientation adjuster 53 has a function of changing the orientation of the array antenna across a predetermined range.

The position derivation by the magnetic field detector 6 in the intra-subject position detection system of the fourth embodiment will be described. First, the reference sensor 50 adjusts the orientation of the array antenna 51 by the orientation adjuster 53 while searching for a direction where the reference sensor 50 can receive the radio signal sent from the magnetic field detector 6. When the orientation set by the orientation adjuster 53 matches with the direction of the magnetic field detector 6, the reference sensor 50 receives the radio signal through the array antenna 51. Then, the received signal strength detecting unit 26 detects the strength of the received radio signal. At the same time, the distance deriving unit 27 derives the distance between the reference position at which the array antenna 51 is positioned and the magnetic field detector 6. Then, information concerning the distance is transmitted to the position deriving unit 28.

On the other hand, the position deriving unit 28 obtains information concerning the orientation at a current time from the orientation adjuster 53. Since the orientation with which the radio signal is received from the magnetic field detector 6 matches with the direction of the magnetic field detector 6, the position deriving unit 28 derives the position of the magnetic field detector 6 based on the orientation and the distance derived by the distance deriving unit 27. Here, the position of the magnetic field detector 6 derived through such process is represented by a three-dimensional polar coordinate. The position deriving unit 28 may convert the three-dimensional polar coordinate into three-dimensional orthogonal coordinate system and output the result through the output unit 23.

The intra-subject position detection system according to the fourth embodiment directly detects the distance between the reference position and the magnetic field detector 6 and the direction of the magnetic field detector 6 to derive the position of the magnetic field detector 6. Therefore, the intra-subject position detection system of the fourth embodiment can realize the position detection of the magnetic field detector 6 accommodating the individual difference in the movement of the subject 1 without performing a complicated calculation.

FIFTH EMBODIMENT

An intra-subject position detection system according to a fifth embodiment will be described. The intra-subject position detection system according to the fifth embodiment has a function of processing a radio signal sent from a capsule endoscope, which is the subject insertable device, using a position information deriving unit.

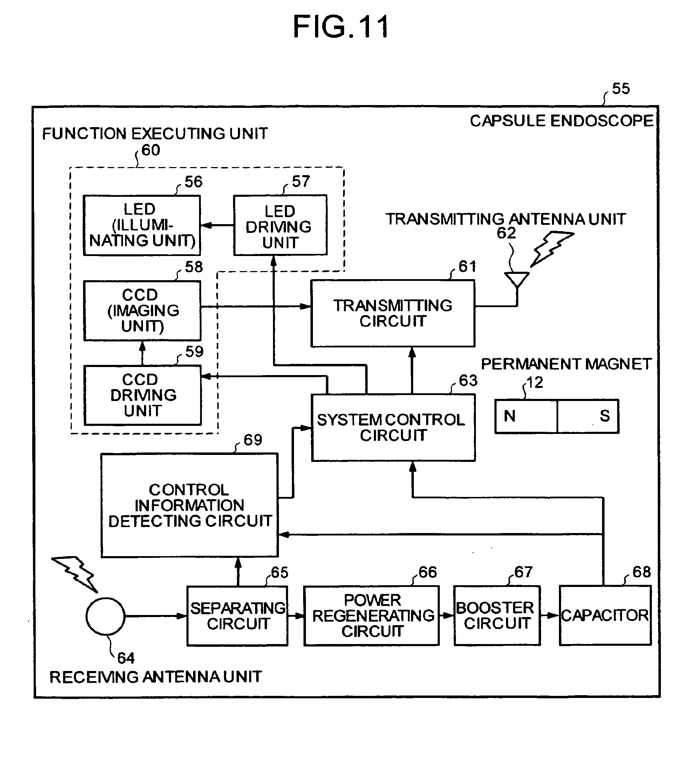

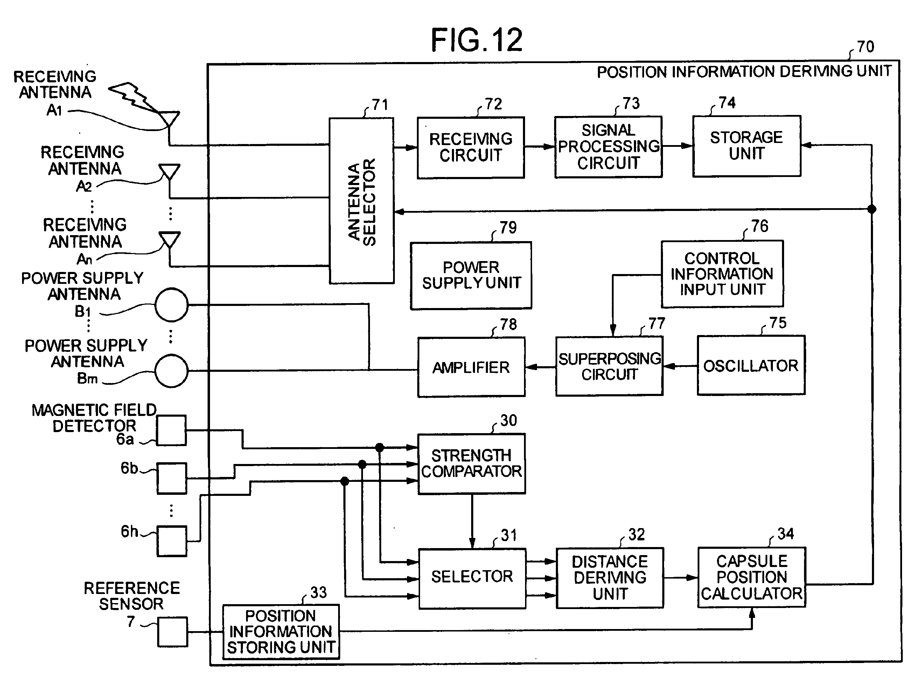

FIG. 11 is a block diagram of a structure of a capsule endoscope in the intra-subject position detection system according to the fifth embodiment. FIG. 12 is a block diagram of a structure of a position information deriving unit in the intra-subject position detection system. The elements which are common to those in the first to fourth embodiments are not shown in the drawings and/or the description thereof will not be repeated.

As shown in FIG. 11, a capsule endoscope 55 includes, in addition to the permanent magnet 12, an LED 56, an LED driving circuit 57, a CCD 58, and a CCD driving circuit 59. The LED 56 functions as an illuminating unit to illuminate an imaging region at the time of imaging inside the subject 1. The LED driving circuit 57 controls a driven state of the LED 56. The CCD 58 functions as an imaging unit to pick up an image of the region illuminated by the LED 56 by receiving a reflected light. The CCD driving circuit 59 controls a driven state of the CCD 58. The LED 56, the LED driving circuit 57, the CCD 58, and the CCD driving circuit 59 are defined collectively as a function executing unit 60 (intra-subject information obtaining unit) that performs a predetermined function.

The capsule endoscope 55 includes a transmitting circuit 61 that generates an RF signal by modulating image data picked up by the CCD 58, a transmitting antenna unit 62 that serves as a radio unit that performs radio transmission of the RF signal output from the transmitting circuit 61, and a system control circuit 62 that controls operations of the LED driving circuit 57, the CCD driving circuit 59, and the transmitting circuit 61.

The capsule endoscope 55 having these elements obtains image data of an examined region illuminated by the LED 56 using the CCD 58 while the capsule endoscope 55 is inside the subject 1. The obtained image data is converted into the RF signal by the transmitting circuit 61 and transmitted via the transmitting antenna unit 62 to the outside.

Further, the capsule endoscope 55 has a structure to receive the radio signal transmitted from a position information deriving unit 70 side. Specifically, the capsule endoscope 55 includes a receiving antenna unit 64 that receives the radio signal sent from the position information deriving unit 70 side, and a separating circuit 65 that separates a power supply signal from the signal received by the receiving antenna unit 64. Further, the capsule endoscope 55 includes a power regenerating circuit 66 that regenerates power from the separated power supply signal, a booster circuit 67 that boosts regenerated power, and a capacitor 68 that accumulates boosted power. The capsule endoscope 55 further includes a control information detecting circuit 69 that detects a content of a travel state information signal from the component separated from the power supply signal by the separating circuit 65, and outputs the detected travel state information signal to the system control circuit 63.

The capsule endoscope 55 having these elements receives the radio signal transmitted from the position information deriving unit 70 side using the receiving antenna unit 64, and separates the power supply signal and the travel state information signal from the received radio signal using the separating circuit 65.

The travel state information signal separated by the separating circuit 65 is supplied as an input to the system control circuit 63 via the control information detecting circuit 69. The system control circuit 63 controls the driven state of the LED 56, CCD 58, and transmitting circuit 61 based on the travel state information. Specifically, when the system control circuit 63 obtains the travel state information which indicates that the capsule endoscope 55 stops moving inside the subject 1, the system control circuit 63 controls the driven state of the CCD 58 and the LED 56 so that the driving of the CCD 58 and the LED 56 temporarily stops in order to prevent the duplicate acquisition of the image data. On the other hand, the power regenerating circuit 66 regenerates power from the power supply signal. The potential of the regenerated power is boosted up to a suitable level for the capacitor 68 by the booster circuit 67. Then, the boosted power is accumulated in the capacitor 68.

A position detecting device of the fifth embodiment will be described with reference to FIG. 12. As shown in FIG. 12, the position detecting device includes, in addition to the structure of the first to fourth embodiments, receiving antennae A1 to An and power supply antennae B1 to Bm. Thus, the position detecting device has a function as a reception unit that receives the radio signal sent from the capsule endoscope 55 and a function as a transmission unit that transmits a predetermined signal to the capsule endoscope 55 by radio.

Firstly, the position information deriving unit 70 has a structure as a reception unit that receives image data of inside the subject. The image data is sent by radio from the capsule endoscope 55. Specifically, the position information deriving unit 70 includes a receiving circuit 72, a signal processing circuit 73, and a storage unit 74. The receiving circuit 72 performs a predetermined processing such as demodulation on the radio signal received by a selected receiving antenna. The signal processing circuit 73 performs necessary processing on the supplied image data. The storage unit 74 stores image data or the like after the image processing.

The storage unit 74 has a function of storing the image data as well as the position information of the capsule endoscope 55 derived by the capsule position calculator 34. Since the intra-subject position detection system of the fifth embodiment has such a structure, the display device 4 can present an image of inside the subject 1 together with an indication of a position of the image pick-up inside the subject 1.

The position information deriving unit 70 has a structure as a transmission unit that generates the power supply signal and the travel state information signal both to be transmitted to the capsule endoscope 55, and transmits the generated signals to the power supply antennae B1 to Bm. Specifically, as shown in FIG. 3, the position information deriving unit 70 includes an oscillator 75, a control information input unit 76, a superposing circuit 77, and an amplifier circuit 78. The oscillator 75 has a function of generating the power supply signal and a function of regulating an oscillating frequency. The control information input unit 76 generates the travel state information signal described later. The superposing circuit 77 superposes the power supply signal and the travel state information signal. The amplifier circuit 78 amplifies the strength of the signal obtained as a result of superposition. The signal obtained after the amplification by the amplifier circuit 78 is sent to the power supply antennae B1 to Bm, and transmitted to the capsule endoscope 55. The position information deriving unit 70 includes a power supply unit 79 that has a predetermined capacitor or an AC power adapter. Each element in the position information deriving unit 70 uses the power supplied from the power supply unit 79 as a driving energy.

As described above, the intra-subject position detection system can use the capsule endoscope as well as the test capsule as the subject insertable device. Further, since the intra-subject position detection system stores the picked-up image data together with the position information of the capsule endoscope 55, a user can easily grasp which region inside the subject 1 corresponds to an image displayed on the display device4.

FIRST EXAMPLE

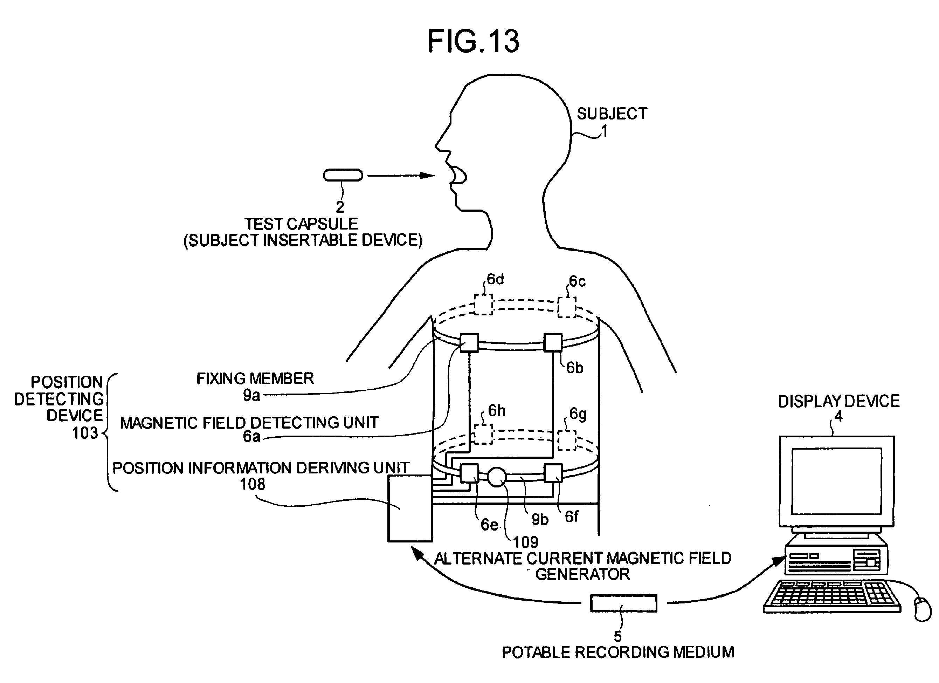

An intra-subject position detection system according to a first example will be described. FIG. 13 is a schematic diagram of an overall structure of the intra-subject position detection system of the first example. As shown in FIG. 13, the intra-subject position detection system includes the test capsule 2, a position detecting device 103, the display device 4, and the portable recording medium 5. The test capsule 2 is introduced into the subject and functions as an example of the subject insertable device. The position detecting device 103 detects the position of the test capsule 2 inside the subject 1. The display device 4 presents position information of the test capsule 2 detected by the position detecting device 103. The portable recording medium 5 serves to deliver information between the position detecting device 103 and the display device 4.

The position detecting device 103 will be described. The position detecting device 103 serves to detect the position of the test capsule 2 inside the subject based on the magnetostatic field output from the test capsule 2. Specifically, the position detecting device 103, as shown in FIG. 13, includes the magnetic field detectors 6a to 6h, the fixing member 9a, the fixing member 9b, a position information deriving unit 108, and an alternating-current (AC) magnetic field generator 109. The magnetic field detectors 6a to 6h detect the strength of the magnetostatic field output from the test capsule 2. The fixing member 9a secures the magnetic field detectors 6a to 6d to the subject. The fixing member 9b secures the magnetic field detectors 6e to 6h to the subject 1. The position information deriving unit 108 derives the position of the test capsule 2 based on the strength of the magnetic field detected by the magnetic field detectors 6a to 6h. The AC magnetic field generator 109 outputs an alternating-current magnetic field for deriving the positions of the magnetic field detectors 6a to 6h.

The magnetic field detectors 6a to 6h serve to detect a magnetic field strength at respective positions where they are positioned. Specifically, the magnetic field detectors 6a to 6h include a Magneto Impedance (MI) sensor. The MI sensor includes a FeCoSiB amorphous wire, for example, as a magneto-sensitive medium. When a high-frequency electric current is applied to the magneto-sensitive medium, a magnetic impedance of the magneto-sensitive medium dramatically changes due to an external magnetic field. This phenomenon is called MI effect. The MI sensor utilizes the MI effect to detect the magnetic field strength. Though other types of sensors may be employed for the magnetic field detectors 6a to 6h, the use of the MI sensor is advantageous in that a particularly highly sensitive detection of the magnetic field strength can be realized.

The fixing members 9a and 9b serve to secure the magnetic field detectors 6a to 6h to the subject 1. Specifically, the fixing members 9a and 9b are formed in ring shape so as to wrap around the chest of the subject 1. The fixing members 9a and 9b are fixed to the chest of the subject 1 in a close contact state.

The AC magnetic field generator 109 serves to output an alternating-current magnetic field to derive the positions of the magnetic field detectors 6a to 6h. The positions of the magnetic field detectors 6a to 6h change in accordance with the movement of the subject 1. Since the alternating-current magnetic field is used for the position derivation of the magnetic field detectors 6a to 6h, it is not preferable that the position of the AC magnetic field generator 109 substantially changes according to the movement of the subject 1. Therefore, the AC magnetic field generator 109 is fixed around the waist of the subject 1. The changes in the position of the waist are practically ignorable. The arranged position of the AC magnetic field generator 109 is not limited to a position close to the waist; for example, the AC magnetic field generator 109 can be arranged near the neck of the subject 1.

Further, the AC magnetic field generator 109 has a function of outputting a reference alternating-current (AC) signal corresponding to the self-induced alternating-current magnetic field to a subtracter 118 described later. Specifically, the reference AC signal is defined as a signal that has an equal frequency to that of the alternating-current magnetic field output from the AC magnetic field generator 109 and that has amplitude corresponding to the strength of the output AC magnetic field.

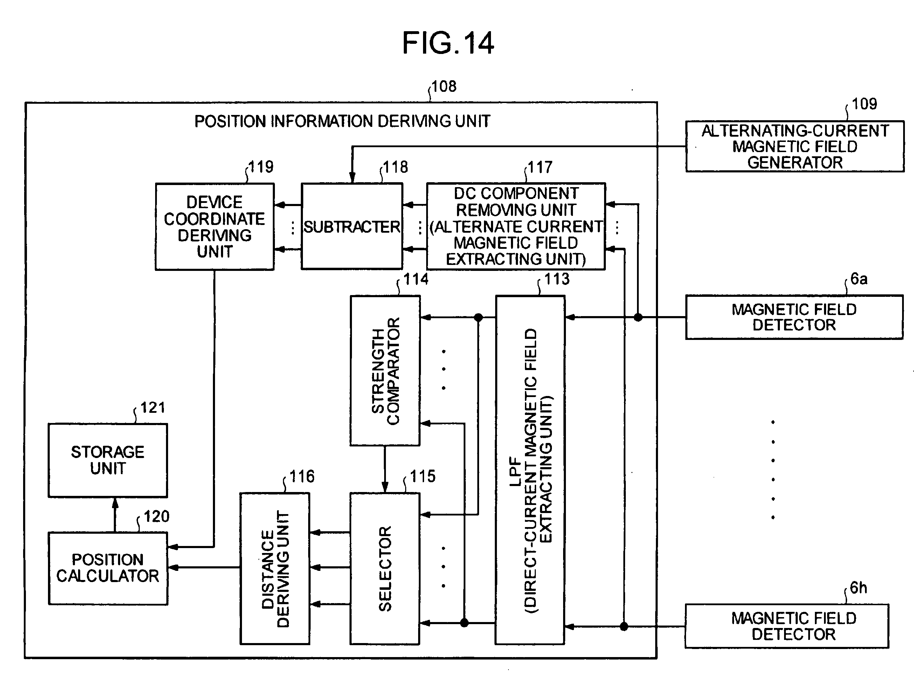

The position information deriving unit 108 will be described. FIG. 14 is a schematic block diagram of a structure of the position information deriving unit. The position information deriving unit 108 has a function of deriving the positions of the magnetic field detectors 6a to 6h. The positions of the magnetic field detectors 6a to 6h change due to the movement of the subject 1. Further, the position information deriving unit 108 has a function of deriving the position of the test capsule 2 based on the derived positions of the magnetic field detectors 6a to 6h and the magnetostatic field generated by the test capsule 2 and detected by the magnetic field detectors 6a to 6h. To realize the two functions, the magnetic field detected by the magnetic field detectors 6a to 6h is divided into two different component systems, and performs predetermined processing in each system.

Specifically, the position information deriving unit 108 includes an element to extract a direct-current (DC) magnetic field component of the detected magnetic field and to derive a distance between the test capsule 2 and the magnetic field detectors 6a to 6h in one system; and includes an element to extract an alternating-current (AC) magnetic field component of the detected magnetic field and to derive positions of the magnetic field detectors 6a to 6h in another system. Further, the position information deriving unit 108 includes an element that derives the position of the test capsule 2 based on the results obtained from the respective systems. Hereinafter, three different types of elements in the position information deriving unit 108 will be described in order.

The position information deriving unit 108 includes, as the element that derives the distance between the magnetic field detectors 6a to 6h and the test capsule 2, a Low Pass Filter (LPF) 113, a strength comparator 114, a selector 115, and a distance deriving unit 116. The LPF 113 passes only the low-frequency component of the supplied detected magnetic field. The strength comparator 114 compares the strength of the magnetic field that passes through the LPF 113. The selector 115 selects magnetic field detected by a part of the magnetic field detectors 6a to 6h based on the result of comparison by the strength comparator 114. The distance deriving unit 116 derives a distance between the magnetic field detector 6 that is selected by the selector 115 and the test capsule 2.

The LPF 113 functions as an example of a direct-current magnetic field extracting unit recited in the appended claims. The LPF 113 serves to pass only the low-frequency component of the magnetic field detected by the magnetic field detectors 6a to 6h. More specifically, the LPF 113 is designed to pass a magnetostatic field component of the detected magnetic field, i.e., only the direct-current magnetic field component. As described above, the permanent magnet 12 of the test capsule 2 has a function of generating the magnetostatic field. The intra-subject position detection system of the sixth embodiment derives the distance between the test capsule 2 and the magnetic field detectors 6a to 6h by performing the operation shown in the equation (1) based on the detected strength of the magnetostatic field. Hence, the alternating-current magnetic field component needs to be removed from the detected magnetic field for the distance derivation. Thus, the LPF 113 is arranged in a previous stage of the distance deriving unit 116.

The strength comparator 114 serves to compare the strength of the direct-current magnetic field component of the magnetic fields detected by the magnetic field detectors 6a to 6h. Specifically, the strength comparator 114 selects three magnetic field detectors from the magnetic field detectors 6a to 6h. The detected magnetic fields of the selected magnetic field detectors have a direct-current magnetic field component with a higher strength. The result of selection is supplied as an output to the selector 115. The selector 115 outputs a direct-current magnetic field component corresponding to the result of selection to the distance deriving unit 116.

The distance deriving unit 116 serves to derive the distance between the reference unit and the test capsule 2 and the distance between the selected device and the test capsule 2 based on the magnetic field strength supplied through the selector 115. Specifically, the distance deriving unit 116 has a function of performing the operation shown by the equation (1) with respect to the input magnetic field strength to derive the distance between the test capsule 2 and the magnetic field detector, magnetic field strength of whose detected magnetic field is detected.

The position information deriving unit 108 includes, as elements that derive the positions of the magnetic field detectors 6a to 6h, a DC component removing unit 117 that removes the direct-current magnetic field component from the detected magnetic field, a subtracter 118 that performs a predetermined subtraction on the magnetic field component whose direct-current magnetic field component is removed, and a device coordinate deriving unit 119 that derives the position coordinates of the magnetic field detectors 6a to 6h based on the result of subtraction.

The DC component removing unit 117 functions as an example of an alternating-current magnetic field extracting unit recited in the appended claims. The DC component removing unit 117 serves to remove the direct-current magnetic field component from the magnetic field detected by the magnetic field detectors 6a to 6h. In the present embodiment, the magnetic field supplied from the alternating-current magnetic field generator 109 for the position derivation of the magnetic field detectors 6a to 6h is an alternating-current magnetic field. Therefore, it is desirable that the direct-current magnetic field component which is irrelevant to the position derivation be removed. Specifically, the DC component removing unit 117 includes a capacitor or the like to remove the direct-current magnetic field component.

The subtracter 118 serves to extract a difference between a reference alternating-current signal supplied from the alternating-current magnetic field generator 109 and an extracted alternating-current magnetic field component of the detected magnetic field. The alternating-current magnetic field component is obtained by the removal of the direct-current magnetic field component by the DC component removing unit 117. Here, the reference alternating-current signal corresponds to the alternating-current magnetic field supplied from the alternating-current magnetic field generator 109. The frequency of the reference alternating-current signal is the same as the frequency of the alternating-current magnetic field, and the amplitude of the reference alternating-current signal corresponds to the strength of the supplied alternating-current magnetic field. Therefore, the result of the operation by the subtracter 118, i.e., the difference derived by the subtracter 118 is a value that indicates a degree of attenuation of the alternating-current magnetic field at the respective positions of the magnetic field detectors 6a to 6h. The device coordinate deriving unit 119 derives the position coordinates of the respective magnetic field detectors 6a to 6h using the value derived by the subtracter 118.

The device coordinate deriving unit 119 functions as an example of a coordinate deriving unit recited in the appended claims. Specifically, the device coordinate deriving unit 119 has a function of deriving a distance between each of the magnetic field detectors 6a to 6h and the alternating-current magnetic field generator 109 based on the strength of the alternating-current magnetic field detected by each of the magnetic field detectors 6a to 6h. Further, the device coordinate deriving unit 119 has a function of deriving the positions of the magnetic field detectors 6a to 6d based on the derived distance and the positional relation among the magnetic field detectors 6a to 6d. Specifically, the device coordinate deriving unit 119 performs the coordinate derivation using the difference between the strength of the alternating-current magnetic field and the reference alternating-current signal output from the alternating-current magnetic field generator 109.

The position information deriving unit 108 includes, as elements that performs the position derivation of the test capsule 2, a position calculator 120 and a storage unit 121. The position calculator 120 derives the position of the test capsule 2 by performing a predetermined operation using the result of derivation by the distance deriving unit 116 and the result of derivation by the device coordinate deriving unit 119. The storage unit 121 stores the position of the test capsule 2 obtained from the operation by the position calculator 120.

The position calculator 120 serves to derive the position of the test capsule 2 by performing a predetermined operation based on the distances between the respective magnetic field detectors 6a to 6h and the test capsule 2. The position calculator 120 also has a function of supplying the result of derivation to the storage unit 121 after deriving the position of the test capsule 2.

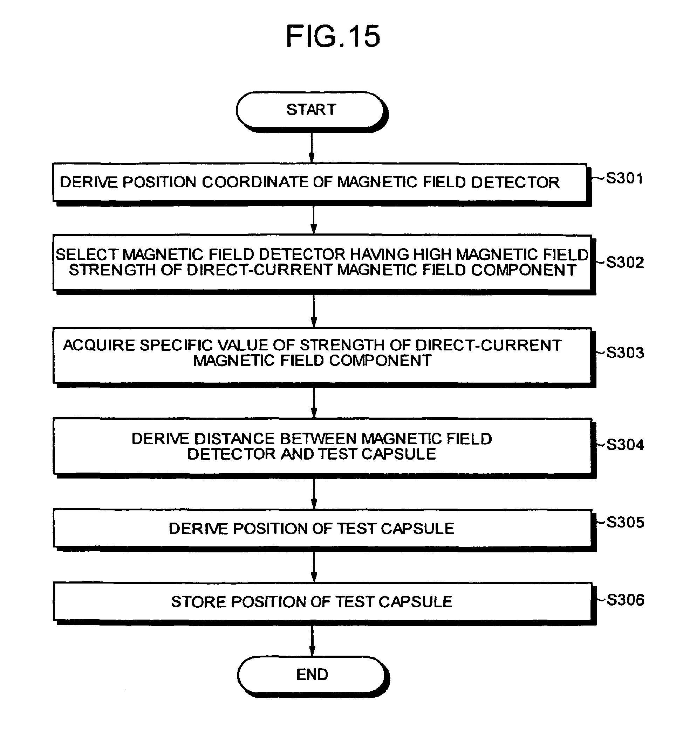

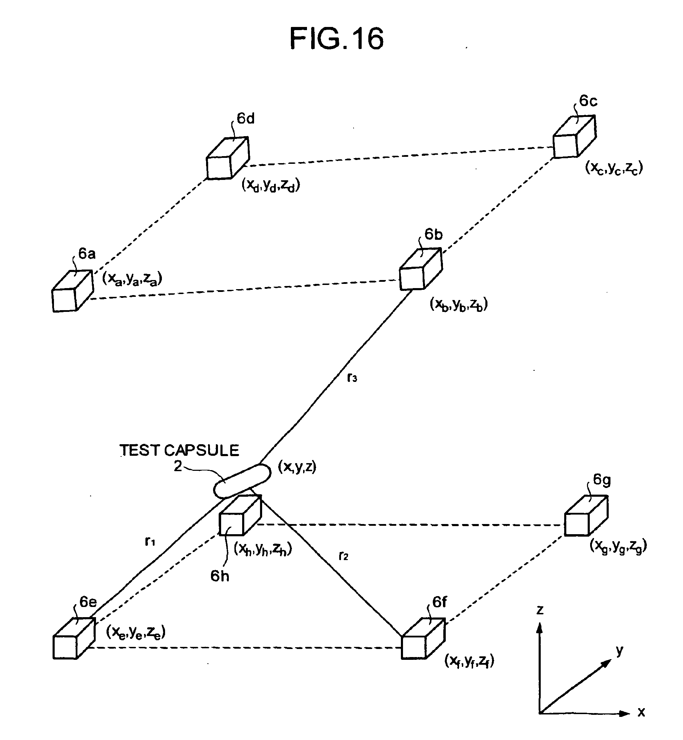

The operation of the position information deriving unit 108 of the first example will be described. FIG. 15 is a flowchart which shows the operation of the position information deriving unit 108; FIG. 16 is a schematic diagram which illustrates the algorithm of the position derivation operation. In FIG. 16, each side of a cube formed by the magnetic field detectors 6a to 6h is supposed to have a length "a". Further, the position of the magnetic field detector 6e which is selected as the reference device is considered to be an origin; a direction from the magnetic field detector 6e toward the magnetic field detector 6f is considered to be the x direction; a direction from the magnetic field detector 6e toward the magnetic field detector 6h is considered to be the y direction; and a direction from the magnetic field detector 6e toward the magnetic field detector 6a is considered to be the z direction. The positions of the magnetic field detectors 6a to 6h are defined based on the xyz coordinate system. The position of the test capsule 2 in the xyz coordinate system is represented as $(x, y, z)$. The operation of the position information deriving unit 108 will be described below with reference to FIGS. 15 and 16 as appropriate.

First, the position information deriving unit 108 derives the position coordinates of the magnetic field detectors 6a to 6h using the device coordinate deriving unit 119 (step S301). Specifically, the device coordinate deriving unit 119 performs a subtraction on the alternating-current magnetic field component which is obtained by the removal of the direct-current magnetic field component by the DC component removal unit 117 from the magnetic field detected by the magnetic field detectors 6a to 6h using the subtracter 118. Thus, the device coordinate deriving unit 119 derives a degree of attenuation of the alternating-current magnetic field supplied from the alternating-current magnetic field generator 109. The device coordinate deriving unit 119 derives the distance between the alternating-current magnetic field generator 109 and the magnetic field detectors 6a to 6h based on the derived degree of attenuation, and derives the positions of the magnetic field detectors 6a to 6h from the result of derivation. In the example shown in FIG. 16, the positions of the magnetic field detectors 6a to 6h are derived as $(x_a, y_a, z_a)$, $(x_b, y_b, z_b)$ ..., as a result of derivation.

Thereafter, the strength comparator 114 in the position information deriving unit 108 selects three magnetic field detectors that detect a strong direct-current magnetic field component from the magnetic field detectors 6a to 6h (step S302). In the example of FIG. 16, the magnetic field detectors 6b, 6e, and 6f are selected.

Then, the distance deriving unit 116 in the position information deriving unit 108 obtains a specific value of the strength of the direct-current magnetic field component (magnetostatic field) at each of the selected magnetic field detectors 6 (step S303). The distance deriving unit 116 then derives the distance between each of the selected magnetic field detectors 6 and the test capsule 2 based on the obtained value (step S304). Specifically, the distance deriving unit 116 derives the distance by solving the equation (1) using the magnetic field strength of the direct-current magnetic field component detected by the selected magnetic field detector 6. In the example of FIG. 16, the distance deriving unit 116 derives the distances $r_1$, $r_2$, and $r_3$ between the test capsule 2 and the respective magnetic field detectors 6e, 6f, and 6b, based on the magnetic field strengths detected at the reference device and the selected devices.

The position information deriving unit 108 obtains the position of the test capsule 2 through the operation by the position calculator 120 (step S305). Specifically, the position calculator 120 performs the operation using the position coordinates of the magnetic field detectors 6a to 6h derived by the device coordinate deriving unit 119 and the distances between the magnetic field detectors and the test capsule 2 derived by the distance deriving unit 116.

For example, the position coordinate (x, y, z) of the test capsule 2 can geometrically be derived based on the positional relation shown in FIG. 16. Specifically, (x, y, z) can be derived by solving the following equations:

$$(x - x_e)^2 + (y - y_e)^2 + (z - z_e)^2 = r_1^2 \qquad (8)$$

$$(x - x_f)^2 + (y - y_f)^2 + (z - z_f)^2 = r_2^2 \qquad (9)$$

$$(x - x_b)^2 + (y - y_b)^2 + (z - z_b)^2 = r_3^2 \qquad (10)$$

In the equations (8) to (10), specific values represented by letters $x_e$, $y_e$, $z_e$, $x_f$, $y_f$, $z_f$, $x_b$, $y_b$, and $z_b$ are derived in step S301 and specific values represented by the letters $r_1$, $r_2$, and $r_3$ are derived in step S304. Hence, only values that remain unknown in the equations (8) to (10) are values represented by the letters x, y, and z that represent the position coordinate of the test capsule 2. The values represented by the letters x, y, and z are derived by solving the equations (8) to (10) by the position calculator 120.

Finally, the storage unit 121 in the position information deriving unit 108 stores the position of the test capsule 2 derived in step S305 (step S306). Specifically, the storage unit 121 stores the position information obtained in step S305 in the portable recording medium 5 since the portable recording medium 5 is attached to the storage unit 121 while the test capsule 2 is inside the subject 1.

The steps from S301 to S306 are repeated at predetermined time intervals. As a result, the portable recording medium 5 stores precise information on the movement of the test capsule 2 inside the subject 1. After the test capsule 2 is discharged outside the subject 1, the portable recording medium 5 is attached to the display device 4. The user can grasp the movement of the test capsule 2 inside the subject based on the recorded results presented on the display device 4. Then, the user determines the position of the stenosis and the presence/absence of the stenosis, for example, in the subject 1.

Advantages of the intra-subject position detection system of the first example will be described. The intra-subject position detection system derives the position of the test capsule 2 based on the magnetostatic field generated by the permanent magnet 12 in the test capsule 2. Different from electromagnetic waves or the like, the magnetostatic field has a characteristic that the strength thereof constantly attenuates regardless of variations in physical parameters such as relative permittivity and magnetic permeability in a region it propagates. Hence, the relation of the equation (1) holds well. Therefore, even when the position to be detected is located inside the space where objects with different physical parameters exist, for example, even when the position inside the human body where various internal organs with different physical parameters exist is to be detected, a highly accurate result can be obtained in comparison with a result obtained by position detection using the electromagnetic waves, for example.

Another advantage of the intra-subject position detection system of the first example is that the pains of the subject 1 can be alleviated at the time of introduction of the test capsule 1 into the subject 1. Due to the above-described reasons, the intra-subject position detection system of the sixth embodiment can suppress the deterioration of the detection accuracy caused by the changes in the surrounding environment of the test capsule 2. Therefore, there is less restriction on the subject 1 at the introduction of the test capsule 2 into the subject compared with an examination using other types of system which requires the subject to refrain from drinking and eating. Hence, the subject 1 can lead a normal life even at the time of examination using the test capsule 2, whereby the pains on the subject 1 at the examination can be alleviated.

Further, the intra-subject position detection system of the first example derives the positions of the magnetic field detectors 6a to 6h. The intra-subject position detection system of the sixth embodiment, having such structure, can accurately derive the position of the test capsule 2 even when the subject 1 moves, for example, to change the positional relation between the fixing member 9b and the fixing member 9a, thereby changing the positions of the magnetic field detectors 6a to 6h.

Still further, in the first example, the alternating-current magnetic field generator 109 is provided for the position derivation of the magnetic field detectors 6a to 6h. The alternating-current magnetic field generator 109 outputs the alternating-current magnetic field, and the position of the magnetic field detectors 6a to 6h are derived based on the detected strength of the alternating-current magnetic field. Since the magnetic field detectors 6a to 6h originally has a function of magnetic field detection for the position detection of the test capsule 2, the intra-subject position detection system of the sixth embodiment does not need to additionally be equipped with a special mechanism for the position derivation by the magnetic field detectors 6a to 6h. Therefore, the intra-subject position detection system of the sixth embodiment can realize even more accurate position detection of the test capsule 2 at a low manufacturing cost.

Still further, in the first example, the alternating-current magnetic field is employed for the position derivation of the magnetic field detectors 6a to 6h. As described above, the test capsule 2 of the sixth embodiment has the permanent magnet 12 that generates the magnetostatic field for the position detection of the test capsule 2. On the other hand, only the alternating-current magnetic field is employed for the position derivation of the magnetic field detectors 6a to 6h. Therefore, the influence of the magnetostatic field generated by the permanent magnet 12 can be eliminated at the position derivation of the magnetic field detectors 6a to 6h.

Here, the alternating-current magnetic field is employed for the position derivation of the magnetic field detectors 6a to 6h. Different from the position detection of the test capsule 2, however, the changes in attenuation rate caused by the objects inside the subject 1 practically do not affect the position derivation. Different from the test capsule 2 that moves through a wide range, i.e., from the esophagus to the large intestine, the magnetic field detectors 6a to 6h do not change the positions thereof significantly even though they move in accordance with the movement of the subject 1. In addition, the objects that are located inside the subject 1 and come between the alternating-current magnetic field generator 109 and the magnetic field detectors 6a to 6h are basically the same even when the positions of the magnetic field detectors change. Therefore the error in position derivation caused by the changes in attenuation rate can be alleviated when, for example, a mechanism is provided to compare the strengths of the radio signals sent from the magnetic field detectors 6a to 6h at the initial state and the strengths of the radio signals at the position detection.


SECOND EXAMPLE

The intra-subject position detection system of the second example will be described. The intra-subject position detection system of the second example includes a capsule endoscope and a position information deriving unit. The capsule endoscope is the subject insertable device and includes a magnetostatic field generator, a predetermined function executing unit, and a radio unit. The position information deriving unit detects the position and the orientation (i.e., direction of the longitudinal axis) of the capsule endoscope inside the subject based on the magnetostatic field generated by the magnetostatic field generator, and selects an antenna that receive radio signals sent from the capsule endoscope from among plural antennae based on the result of detection.

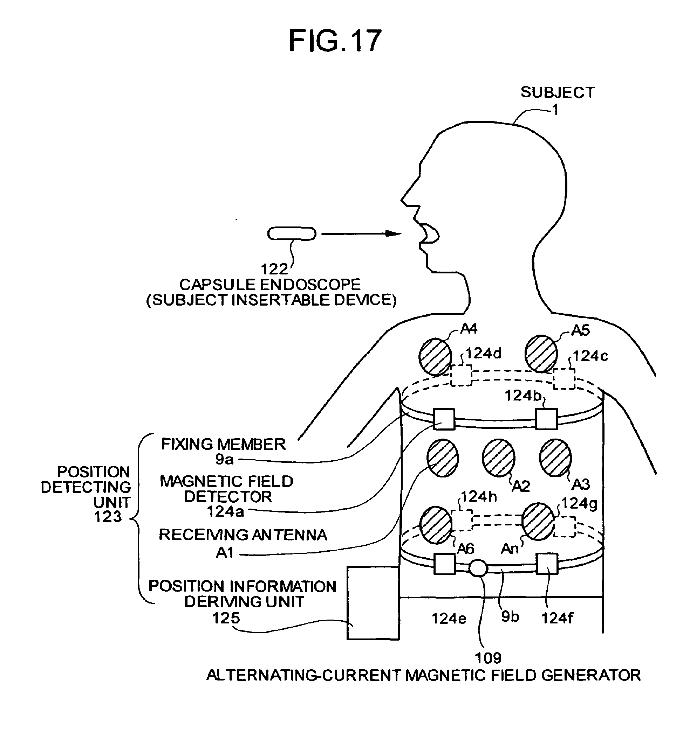

FIG. 17 is a schematic diagram showing an overall structure of the intra-subject position detection system of the second example. As shown in FIG. 17, the intra-subject position detection system of the seventh embodiment includes a capsule endoscope 122 which is an example of the subject insertable device, and a position detecting device 123. In FIG. 17, elements corresponding to the display device 4 and the portable recording medium 5 of the first example are not shown, though exclusion from the drawing should not be taken as to intend elimination of these elements from the second example. In the intra-subject position detection system of the second example, the elements denoted by the same reference characters and the names as those in the first example have the same structure and function as those in the first example, if not specified otherwise below.

The position detecting device 123, as shown in FIG. 17, includes magnetic field detectors 124a to 124h, the fixing members 9a and 9b, the receiving antennae A1 to An, and a position information deriving unit 125. The fixing members 9a and 9b secure the magnetic field detectors 124a to 124h to the subject 1. The receiving antennae A1 to An receive the radio signals sent from the capsule endoscope 122. The position information deriving unit 125 processes the information obtained by the magnetic field detectors 124a to 124h and the receiving antennae A1 to An, to obtain the position information of the capsule endoscope 122 inside the subject 1.

The magnetic field detectors 124a to 124h each serve to detect strength and direction of the magnetic field at the position thereof. Specifically, the magnetic field detectors 124a to 124h includes an MI sensor or the like that has a function of detecting the strength and direction of the magnetic field. The magnetic field detectors 6a to 6h of the sixth embodiment are designed to detect only the magnetic field strength. The magnetic field detectors 124a to 124h of the seventh embodiment, however, are designed to detect not only the strength but also the direction, since the intra-subject position detection system of the seventh embodiment detects the position as well as the orientation of the subject insertable device (capsule endoscope 122).

The receiving antennae A1 to An serve to receive the radio signal sent from the capsule endoscope 122. As described later, the capsule endoscope 122 of the seventh embodiment has a function of obtaining an image inside the subject 1 and transmitting the image by radio to the outside. The receiving antennae A1 to An receive the radio signal sent from the capsule endoscope 122 and outputs the received radio signal to the position information deriving unit 125. Specifically, the receiving antennae A1 to An includes a loop antenna and a fixer that fixes the loop antenna to the subject 1, for example. The receiving antennae A1 to An may be configured in such a manner that all of the receiving antennae A1 to An receive the radio signal when the radio signal is transmitted from the capsule endoscope 122. In the seventh embodiment, however, only the receiving antenna which is most suitable for the reception is selected by an antenna selector 149 described later from the plural receiving antennae A1 to An.

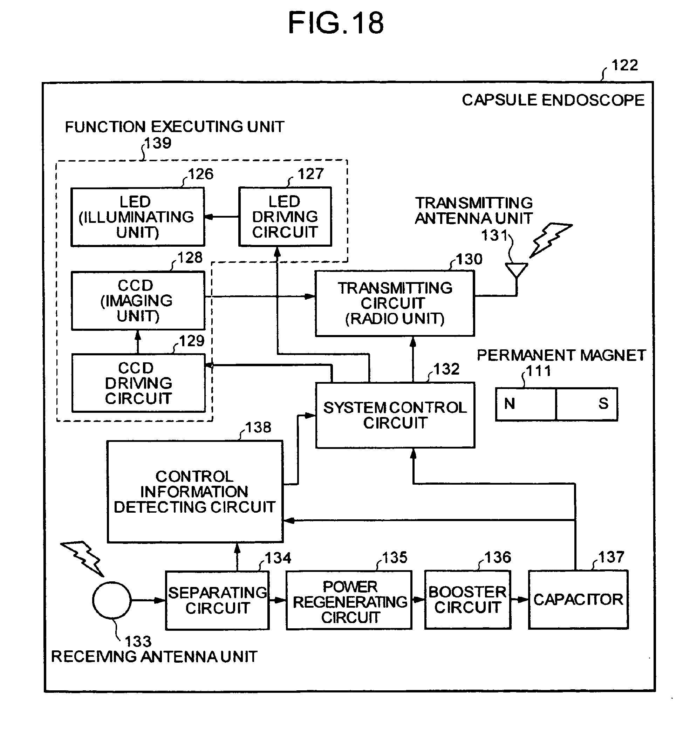

FIG. 18 is a block diagram showing a structure of the capsule endoscope 122. The capsule endoscope 122, similarly to the test capsule 2 of the first embodiment, includes the permanent magnet 111 as the magnetostatic field generator. Further, the capsule endoscope 122 includes an LED 126, an LED deriving circuit 127, a CCD 128, and a CCD driving circuit 129. The LED 126 functions as an illuminating unit that illuminates an imaging region at the image pick-up of inside the subject 1. The LED driving circuit 127 controls a driven-state of the LED 126. The CCD 128 functions as an imaging unit that obtains an image of the region illuminated by the LED 126 by receiving the reflected light therefrom. The CCD driving circuit 129 controls a driven-state of the CCD 128. Here, the LED 126, the LED driving circuit 127, the CCD 128, and the CCD driving circuit 129 are collectively defined as a function executing unit 139 that performs a predetermined function as an intra-subject information obtaining unit that serves to obtain predetermined information of the inside of the subject 1.

Further, the capsule endoscope 122 includes a transmitting circuit 130, a transmitting antenna unit 131, and a system control circuit 132. The transmitting circuit 130 modulates the image data obtained by the CCD 128 and generates an RF signal. The transmitting antenna unit 131 serves as a radio unit that transmits the RF signal supplied from the transmitting circuit 130 by radio. The system control circuit 132 controls the operations of the LED driving circuit 127, the CCD driving circuit 129, and the transmitting circuit 130.

The capsule endoscope 122 having the above-described structure obtains the image data of an examined region which is illuminated by the LED 126 using the CCD 128 while the capsule endoscope 122 is inside the subject 1. The transmitting circuit 130 converts the obtained image data into the RF signal and transmits the RF signal through the transmitting antenna unit 131 to the outside.

The capsule endoscope 122 further includes a receiving antenna unit 133, and a separating circuit 134. The receiving antenna unit 133 receives the radio signal sent from a position detecting device 123 side. The separating unit 134 separates the power supply signal from the signal received at the receiving antenna unit 133. Further, the capsule endoscope 122 includes a power regenerating circuit 135, a booster circuit 136, and a capacitor 137. The power regenerating circuit 135 regenerates power from the power supply signal separated from the received signal. The booster circuit boosts the regenerated power. The capacitor accumulates the boosted power. The capsule endoscope 122 includes a control information detecting circuit 138 that detects a content of a control information signal from a component separated from the power supply signal at the separating circuit 134, and outputs the detected control information signal to the system control circuit 132. The system control circuit 132 also has a function of distributing a driving power supplied from the capacitor 137 to other elements.

The capsule endoscope 122 having the above described structure receives the radio signal sent from the position detecting device 123 side at the receiving antenna unit 133. Then the separating circuit 134 separates the power supply signal and the control information signal from the received radio signal. The control information signal separated by the separating circuit 134 is supplied to the system control circuit 132 via the control information detecting circuit 138. The control information signal is used for the drive control of the LED 126, the CCD 128, and the transmitting circuit 130. On the other hand, the power supply signal is used for regeneration of power by the power regenerating circuit 135. The potential of the regenerated power is raised up to a suitable level for the capacitor 137. Then, the power is accumulated in the capacitor 137.

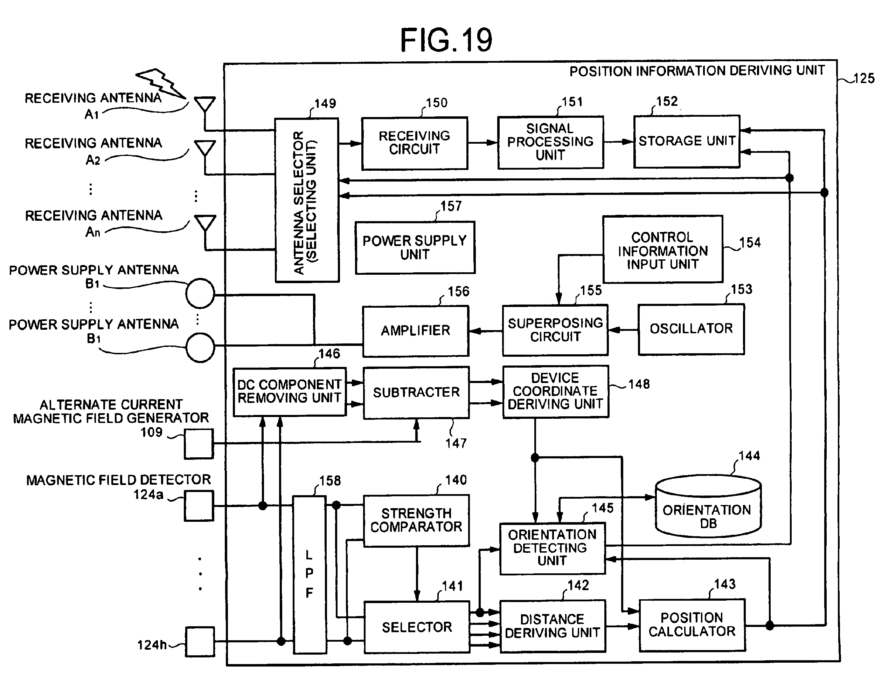

The structure of the position information deriving unit 125 will be described. FIG. 19 is a block diagram of a structure of the position information deriving unit 125. The position information deriving unit 125 of includes, as elements that detect the position of the capsule endoscope 122 inside the subject 1, an LPF 158, a strength comparator 140, a selector 141, a distance deriving unit 142, and a position calculator 143. Since the magnetic field detectors 124a to 124h output not only the magnetic field strength but also the magnetic field direction to the position information deriving unit 125, the strength comparator 140 extracts the magnetic field strength among the supplied information from the magnetic field detectors 124a to 124h for the selection of the reference device. The distance deriving unit 142, on the other hand, extracts the magnetic field strengths received from the reference device and the selected device for the derivation of the distance. In the above-described point, the second example is different from the first example. The operation of position detection of the capsule endoscope 122 in the second example is substantially the same with that in the first example, and the detailed description thereof will not be repeated.

The position information deriving unit 125, similarly to the second example, includes a DC component removing unit 146, a subtracter 147, and a device coordinate deriving unit 148. The DC component removing unit 146 serves to derive the positions of the magnetic field detectors 124a to 124h. The device coordinate deriving unit 148 and the like further has a function of performing a predetermined process by extracting only the magnetic field strength based on the result of detection at the magnetic field detectors 124a to 124h in accordance with the function of the magnetic field detectors 124a to 124h which similarly detect the magnetic field direction.

Further, the position information deriving unit 125 includes an orientation database 144 and an orientation detecting unit 145. The orientation database 144 is employed for the detection of the orientation of the capsule endoscope 122 as described later. The orientation detecting unit 145 detects the orientation of the capsule endoscope 122 based on the magnetic field strength which is detected at a predetermined magnetic field detector and output from the selector 141. The orientation database 144 stores in advance the strength of the magnetic field to be detected by the magnetic field detector 124 and the orientation of the capsule endoscope 122 with respect to the positional relation between the magnetic field detector 124 and the capsule endoscope 122. The specific operation of the orientation database 144 and the orientation detecting unit 145 will be described in detail later.

The 5 position information deriving unit 125 has a function as a reception unit that receives the image data which is of the inside of the subject 1 and sent from the capsule endoscope 122 by radio. Specifically, the position information deriving unit 125 includes an antenna selector 149, a receiving circuit 150, a signal processing unit 151, and a storage unit 152. The antenna selector 149 selects an antenna to be used for data reception from among the receiving antennae A1 to An. The receiving circuit 150 performs predetermined processing such as demodulation on a radio signal received by the selected receiving antenna, extracts the image data obtained by the capsule endoscope 122 from the radio signal, and outputs the extracted image data. The signal processing unit 151 performs necessary processing on the supplied image data. The storage unit 152 stores the image data after the necessary image processing.

The antenna selector 149 serves to select the receiving antenna which is most suitable for the reception of radio signal sent from the capsule endoscope 122. Specifically, the antenna selector 149 grasps the positions of the receiving antennae A1 to An in advance and receives the information that is related with the position of the capsule endoscope 122 and is derived by the position calculator 143 and the information that is related with the orientation of the capsule endoscope 122 and is derived by the orientation detecting unit 145. Therefore, the antenna selector 149 has a function of selecting the receiving antenna which is supposed to have the most favorable reception sensitivity in terms of the position and the orientation of the capsule endoscope 122 and supplying the radio signal received by the selected receiving antenna to the receiving circuit 150.

The storage unit 152 has a function of storing the image data supplied from the signal processing unit 151 and the position and orientation of the capsule endoscope 122 at the time of pick-up of the supplied image data in association with each other. Specifically, the position information deriving unit 125 has such a structure that the information obtained by the position calculator 143, the orientation detecting unit 145, and the signal processing unit 151 are supplied to the storage unit 152 as shown in FIG. 19. The storage unit 152 has a function of storing the supplied information in association with each other. As a result, the storage unit 152 stores the image data of a predetermined region inside the subject 1 and the position and orientation of the capsule endoscope 122 at the time of pick-up of the image data in association with each other.

The position information deriving unit 125 further has a function of generating the power supply signal or the like to be sent to the capsule endoscope 122 and outputting the generated signal to the power supply antennae B1 to Bm. Specifically, the position information deriving unit 125 includes an oscillator 153, a control information input unit 154, a superposing circuit 155, and an amplifier 156. The oscillator 153 has a function of generating the power supply signal and regulating the oscillating frequency. The control information input unit 154 generates a control information signal for the control of the driven-state of the capsule endoscope 122. The superposing circuit 155 superposes the power supply signal and the control information signal. The amplifier 156 amplifies the strength of the superposed signal. The signal obtained by amplification by the amplifier 156 is sent to the power supply antenna B1 to Bm, which transmit the supplied signal to the capsule endoscope 122. The position information deriving unit 125 includes a power supply unit 157 which is provided with a predetermined capacitor or an AC power adapter. Each element of the position information deriving unit 125 employs the power supplied from the power supply unit 157 as the driving energy.

Significance of the detection of the orientation of the capsule endoscope 122 and the content of the orientation detecting operation in the intra-subject position detection system in the second example will be described. As described above, in the intra-subject position detection system according to the second example, the capsule endoscope 122 has a predetermined intra-subject information obtaining unit. The information obtained by the intra-subject information obtaining unit is sent to the position detecting device 123 side by radio. Therefore, the position detecting device 123 has plural receiving antennae A1 to An for the reception of the transmitted radio signal. The receiving antenna which is most suitable for the reception is selected from the plural receiving antennae A1 to An by the antenna selecting unit 149.

The algorithm for selection of the most suitable receiving antenna from the plural receiving antennae A1 to An may be determined based primarily on the positional relation between the receiving antenna and the capsule endoscope 122. For example, it is possible to derive the position of the capsule endoscope 122 using a position detecting mechanism similar to the one in the sixth embodiment and to use the receiving antenna which is closest to the derived position, if the radio signal sent from the capsule endoscope 122 is assumed to be attenuated as a function of distance therebetween.

When the radio signal is received from the capsule endoscope, however, it is not always appropriate to select the receiving antenna based solely on the positional relation with the antenna. Since the transmitting antenna unit 131 used for the radio transmission from the capsule endoscope 122 is formed of a loop antenna, for example, the transmitting antenna unit 131 does not send the radio signal with an equal strength in every direction. Instead, the transmitting antenna unit 131 sends the radio signal with a certain degree of directivity. Therefore, the receiving antenna which is the most suitable for the reception of the radio signal from the capsule endoscope preferably is determined not solely based on the positional relation with the capsule endoscope, but also in consideration of the directionality of the radio signal sent from the transmitting antenna unit 131. Since the transmitting antenna unit 131 is secured inside the capsule endoscope 122, to know the orientation of the capsule endoscope 122 inside the subject is important for the detection of the directivity of the transmitted radio signal. In view of the above, the second example includes a mechanism for detecting the position of the capsule endoscope 122 inside the subject similarly to the sixth embodiment; and further includes the orientation database 133 and the orientation detecting unit 145, so that the orientation of the capsule endoscope 122 can be detected.

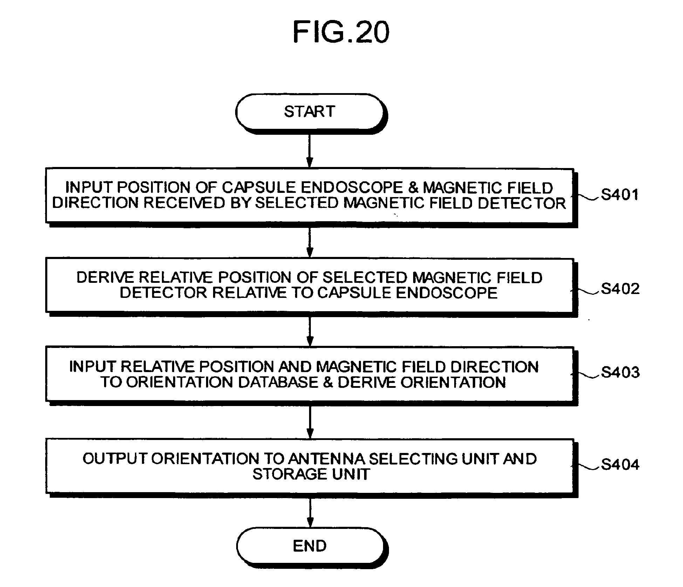

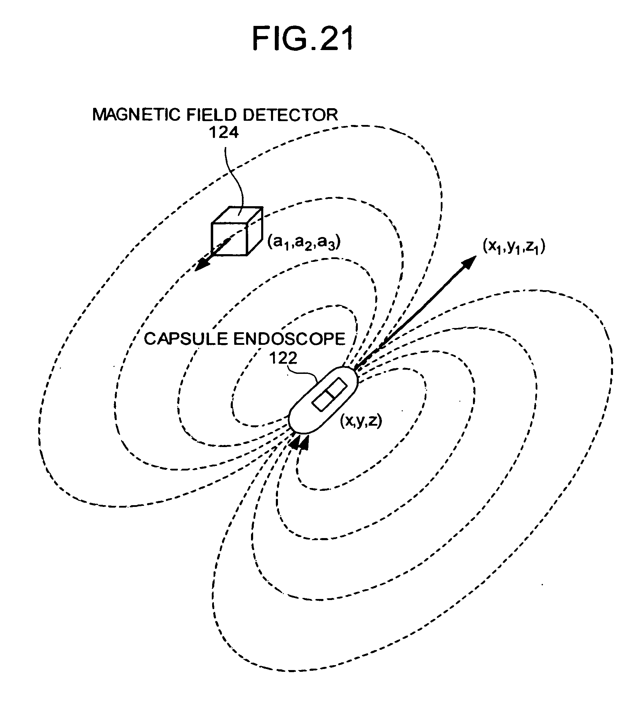

FIG. 20 is a flowchart which shows the operation by the orientation detecting unit 145 to detect the orientation of the capsule endoscope 122. FIG. 21 is a schematic diagram which shows the relation between the orientation of the capsule endoscope 12 and the magnetic field detectors 124. The operation of the orientation detecting unit 145 will be described with reference to FIGS. 20 and 21 as appropriate.

Firstly, the orientation detecting unit 145 receives the position of the capsule endoscope 122 and the direction of the magnetic field detected by the magnetic field detector 124 which is selected from the plural magnetic field detectors 124a to 124h (step S401). Any algorithm can be employed as the selection algorithm of the magnetic field detector 124. In the second example, however, the magnetic field detector 124 with the strongest detected magnetic field is selected, for example. In an example of FIG. 21, the orientation detecting unit 145 grasps the magnetic field direction which is represented by the coordinate $(a_1, a_2, a_3)$ of the selected magnetic field detector 124 and the direction vector shown by an arrow.

The orientation detecting unit 145 derives a relative position of the magnetic field detector 124 selected in step S401 with respect to the capsule endoscope 122 (step S402). Specifically, the orientation detecting unit 145 receives the position of the capsule endoscope 122 derived by the position calculator 143, and derives the relative coordinate of the magnetic field detector 124 selected in step S401 with respect to the capsule endoscope 122. In the example of FIG. 21, the relative position coordinate $(a_1 - x, a_2 - y, a_3 - z)$ of the magnetic field detector 124 with respect to the position of the capsule endoscope 122 as the origin is derived based on the coordinate $(a_1, a_2, a_3)$ of the magnetic field detector 124 and the coordinate $(x, y, z)$ of the capsule endoscope 122.

Thereafter, the orientation detecting unit 145 inputs the magnetic field direction supplied in step S401 and the relative position of the magnetic field detector 124 selected in step S402 to the orientation database 144 to obtain data concerning the orientation of the capsule endoscope 122 (step S403). As shown in FIG. 21, the direction of the magnetostatic field generated by the permanent magnet in the capsule endoscope 122 is uniquely determined according to the orientation of the capsule endoscope 122 and the position relative to the capsule endoscope 122 and the position relative to the capsule endoscope 122. The orientation database 144 stores the orientation of the capsule endoscope 122, the relative coordinate with respect to the capsule endoscope 122, and the direction of the magnetostatic field in the relative coordinate in association with each other. Therefore, when the orientation database 144 receives the relative coordinate of the magnetic field detector 124 and the direction of the detected magnetostatic field, the orientation of the capsule endoscope 124 can be extracted from the orientation database 144. In the example of FIG. 21, the orientation of the capsule endoscope 122 is derived to be (x1, y1, z1) based on the result of output from the orientation database 144.

Finally, the orientation detecting unit 145 outputs the obtained data on the orientation of the capsule endoscope 122 to the antenna selector 149 and the storage unit 152 (step S404). The antenna selector 149 selects the receiving antenna most suitable for the reception based on the data on the orientation and the information that is related with the position and output from the position calculator 143. The storage unit 152 stores the orientation of the capsule endoscope 122 at a predetermined time in association with the image data and the position information of the capsule endoscope 122.

Advantages of the intra-subject position detection system according to the second example will be described. In the intra-subject position detection system of the second example, the capsule endoscope 122 includes the permanent magnet 111 similarly to that of the first example, and the position of the capsule endoscope 122 is detected based on the magnetostatic field generated by the permanent magnet 111. As described earlier, the magnetostatic field has a characteristic of constantly attenuating according to the distance regardless of the difference in relative permittivity, electric conductivity, or the like of the internal organs or the like inside the subject 1. Therefore, when the magnetostatic field is employed for the position detection, more accurate position detection of the capsule endoscope 122 can be realized compared with the position detection using the radio signal.

Further, the intra-subject position detection system of the second example detects the orientation of the capsule endoscope 122 based on the magnetostatic field generated by the permanent magnet 111. The magnetostatic field generated by the permanent magnet 111 is hardly affected by the presence of objects inside the subject 1. In addition, the magnetic field direction at a predetermined position can be substantially uniquely determined based on the orientation of the capsule endoscope 122 and the relative position with respect to the capsule endoscope 122. Hence, when the distribution of orientations of the magnetostatic field generated by the permanent magnet 111 is previously derived and stored in the orientation database 144, and the orientation database 144 is referred based on the information obtained by the magnetic field detector 124, the orientation of the capsule endoscope 122 can be detected accurately.

Further, the intra-subject position detection system of the second example detects the orientation of the capsule endoscope 122 based on the magnetostatic field similarly to the position detection. Hence, the intra-subject position detection system can be realized by a simplified structure. The intra-subject position detection system of the second example does not need to add new elements to the capsule endoscope 122 to realize the function of detecting the orientation of the capsule endoscope 122, whereby a small low-cost position information detection system can be built.

Further, in the intra-subject position detection system of the second example, the receiving antenna is selected by the antenna selector 149 based on the derived position and orientation of the capsule endoscope 122. The sensitivity of reception of the radio signal by the receiving antenna is dependent on the distance between the capsule endoscope 122 and the receiving antenna and the directivity of the transmitting antenna unit 131 provided in the capsule endoscope 122. Therefore, in the intra-subject position detection system, an accurate selection of the receiving antenna is realized since the selection is based on the position and orientation of the capsule endoscope 122, whereby a position information detection system which can constantly receive the radio signal sent from the capsule endoscope 122 with high sensitivity can be realized.

Further, in the intra-subject position detection system of the second example, the picked-up image data of the inside of the subject 1 and the derived position and orientation of the capsule endoscope 122 are stored in the storage unit 152. Therefore, the image data obtained by the capsule endoscope 122 and the derived position and orientation of the capsule endoscope at the time of image pick-up can be stored in association with each other. When the image data is displayed on the display device 4, only the image data corresponding to a predetermined range in the subject 1 can be displayed. In other words, the user can display only the image data of an interest region, e.g., of a small intestine, rather than displaying all the image data on the display device 4. Thus, a position information detection system which is convenient for doctors or the like can be realized.

The present invention is described with respect to the first to fifth embodiments. The present invention, however, is not limited to the embodiments described above, and those skilled in the art can reach various examples, modified example, and applications. For example, in the fifth embodiment, the function executing unit 60 includes the CCD 58 and the like as the imaging unit and the LED 56 and the like as the illuminating unit. The function executing unit, however, can alternatively be a unit that obtains intra-subject information related with pH, temperature, or the like inside the subject 1. Further, the subject insertable device may include a transducer and obtain an ultrasound image of the inside of the subject 1. Further, the subject insertable device may obtain plural pieces of information from various pieces of intra-subject information.

Further, the number of magnetic field detectors 6 does not need to be limited in the first and second examples. In a most simplified structure, the intra-subject position detection system may include a single magnetic field detector 6. Such structure is possible because: the subject insertable device, such as the test capsule 2 and the capsule endoscope 122, does not move freely inside the subject 1; rather, the subject insertable device moves along a route, which is fixed to a certain degree. For example, the subject insertable device moves through predetermined internal organs such as esophagus, stomach, small intestine, and large intestine. Therefore, the travel route of the subject insertable device can be known in advance to a certain degree. Then, the position of the subject insertable device can be detected based on the travel route information which is previously obtained and the strength of the magnetostatic field detected by the single magnetic field detector.

Similarly, in the second example, for example, the orientation of the capsule endoscope 122 may be derived by the plural magnetic field detectors 124. Specifically, orientation is derived by each of the plural magnetic field detectors 124 in the above describe manner. Then, an average of the derived orientations is found. It is preferable to provide such a structure to enable more accurate derivation of the orientation. This similarly applies to the position detection of the subject insertable device. The position detection may be performed plural times by different combinations of the magnetic field detectors 6 or the like, and an average of the obtained positions may be found.

Further, in the second example, the function executing unit 139 includes the CCD 128 and the like as the imaging unit and the LED 126 and the like as the illuminating unit. The function executing unit, however, may additionally include a unit to obtain information concerning pH and temperature inside the subject 1. Further, the subject insertable device may include a transducer so as to obtain ultrasound images of the inside of the subject 1. Further, the subject insertable device may have a structure to obtain plural pieces of information among the various types of intra-subject information.

The radio signal outpu5 t from the power supply antennae B1 to Bm does not necessarily be a superposed signal of the control information signal and the power supply signal. Further, the intra-subject position detection system may have a structure in which the position detecting device does not perform radio transmission to the capsule endoscope. Still further, the power supply signal may be superposed with a signal other than the control information signal. Still further, the position detecting device 123 may perform solely the reception of the radio signal from the capsule endoscope. The capsule endoscope may include a storage unit so that the information stored in the storage unit is read out after the capsule endoscope is discharged from the subject 1.

In the second example, the selection of the power supply antennae B1 to Bm is not particularly described. Similarly to the selection of the receiving antennae A1 to An, the most suitable power supply antenna may be selected for the radio transmission based on the position and the orientation of the capsule endoscope 122. Specifically, it is possible to send the radio signal only from an antenna which corresponds to the orientation or the like of the receiving antenna unit 133 provided in the capsule endoscope 122 by selecting such an antenna based on the orientation or the like of the capsule endoscope 122 rather than sending the radio signal equally from all the power supply antennae.

The first and second examples may further include plural alternating-current magnetic field generators. Such structure allows the position derivation of the magnetic field detector 6a or the like based only on the distance from the alternating-current magnetic field generators as well as the position detection of the capsule endoscope 122.

## INDUSTRIAL APPLICABILITY

The intra-subject position detection system according to the present invention is suitable for the position detection of the subject insertable device which is introduced into the subject, and more particularly, suitable to realize an accurate position detection without being affected by the presence of internal organs or the like inside the subject.

## Claims

1. An intra-subject position detection system, comprising:

a subject insertable device (2; 55) adapted to be introduced into a subject (1) and move through the subject (1); and
a position detecting device (3) adapted to be arranged outside the subject (1) and obtain position information of the subject insertable device (2; 55) inside the subject (1), wherein
the subject insertable device (2; 55) includes a magnetic field generator (12) adapted to generate a magnetostatic field, wherein
the position detecting device (3) includes
a plurality of magnetic field detector units (6a to 6h; 36a to 36h) adapted to be arranged on the subject (1) at a time of use and detect strengths of the magnetostatic field output from the magnetic field generator (12),
a reference sensor (7; 37; 41; 50) adapted to derive positions of the magnetic field detector units (6a to 6h; 36a to 36h) relative to the reference sensor (7; 37; 41; 50), and
a position information deriving unit (10; 70) adapted to derive a position of the subject insertable device (2; 55) inside the subject (1) based on the magnetic field strengths detected by the magnetic field detector units (6a to 6h; 36a to 36h) and the positions of the magnetic field detector units (6a to 6h; 36a to 36h) detected by the reference sensor (7; 37; 41; 50), wherein
the position detecting device includes a plurality of first radio units (16) fixed tao the magnetic field detector units (6a to 6h; 36a to 36h), respectively, wherein
the reference sensor (7; 37; 41; 50) includes:

a second radio unit (19; 25, 42 to 45; 52) adapted to receive radio signals transmitted from the first radio units (16); and
a distance deriving unit (27) adapted to derive distances between the reference sensor (7; 37; 41; 50) and the positions of the magnetic field detector units (6a to 6h; 36a to 36h) based on received signal strengths of the radio signals at the second radio unit (19; 25, 42 to 45; 52), and wherein,
the position information deriving unit (10; 70) is adapted to acquire and store information related to the positions of the magnetic field detector units (6a to 6h; 36a to 36h), then detect the strengths of the magnetostatic field by the magnetic field detector units (6a to 6h; 36a to 36h), derive distances between the subject insertable device (2; 55) and the magnetic field detector units (6a to 6h; 36a to 36h) based on the detected strengths, and derive the position of the subject insertable device (2; 55) based on the distances and the positional information of the magnetic field detector units (6a to 6h; 36a to 36h).

2. The intra-subject position detection system according to claim 1, wherein
the reference sensor (7; 37; 41; 50) is adapted to derive the distances between the reference sensor (7; 37; 41; 50) and the positions of the magnetic field detector units (6a to 6h; 36a to 36h), and derive the positions of the magnetic field detector units (6a to 6h; 36a to 36h) relative to the reference sensor (7; 37; 41; 50) based on the distances derived.

3. The intra-subject position detection system according to claim 1, wherein the reference sensor (7; 37; 41; 50) further includes:

a position information database (22) that is adapted to store correspondence between the positions of the magnetic field detector units (6a to 6h) and the distances between the reference sensor (7; 37; 41; 50) and each of the magnetic field detector units (6a to 6h), and
a position deriving unit (28) that is adapted to extract positions associated with the distances derived by the distance deriving unit (27) from the correspondence stored in the position information database (22).

4. The intra-subject position detection system according to claim 1, wherein:

the second radio unit includes a plurality of receiving antennas (42, 43, 44, 45) corresponding to a plurality of reference positions, respectively, the receiving antennas (42, 43, 44, 45) being adapted to receive the radio signals transmitted from the first radio units (16),
the reference sensor (41) further comprises:

a selector (45) adapted to sequentially output the radio signal received through the receiving antennae (42, 43, 44, 45),
a receiving circuit (25) adapted to extract strengths of each of the radio signals sequentially outputted by the selector (45), and
the distance deriving unit (27) is adapted to derive distances between each of the reference positions and one of the positions of the magnetic field detector units (6a to 6h) for each of the magnetic field detector units (6a to 6h) based on the strengths extracted by the receiving circuit (25), to derive the positions of the magnetic field detector units (6a to 6h).

5. The intra-subject position detection system according to claim 1,
wherein the reference sensor (50) further includes
an array antenna (51) adapted to detect a direction of the magnetic field detector units (6a-6h, 36a-36h),
an orientation adjusting unit (53) that is adapted to adjust an orientation direction of the array antenna (51), the orientation direction being a direction in which the array antenna (51) matches with the direction of the magnetic field detector units (6a to 6h; 36a to 36h), and
the reference sensor (50) is adapted to derive the positions of the magnetic field detector units (6a to 6h) based on the distances derived by the distance deriving unit (27) and the orientation direction adjusted by the orientation adjusting unit (53).

6. The intra-subject position detection system according to claim 1, wherein
the second radio unit (19) is adapted to transmit the radio signal to each of the first radio units (16) in a time-multiplexed manner.

7. The intra-subject position detection system according to claim 1, wherein
the second radio unit (19) is adapted to transmit the radio signal of a different frequency to each of the first radio units (16).

8. The intra-subject position detection system according to claim 1, wherein
the subject insertable device (55) further includes
a predetermined intra-subject information obtaining unit (60) adapted to obtain information inside the subject (1) as intra-subject information when the subject insertable device (55) is introduced into the subject (1) and move through the subject (1), and
a radio transmission unit (61, 62) adapted to transmit the intra-subject information obtained by the intra-subject information obtaining unit (60) by radio, and wherein

the position detecting device (3) further includes
a receiving unit (A1 to An, 71, 72) adapted to receive a radio signal containing the intra-subject information, the radio signal being sent from the radio transmission unit (61, 62).

**9.** The intra-subject position detection system according to claim 8, wherein
the intra-subject information obtaining unit (60) includes
an illuminating unit (56) adapted to illuminate an inside of the subject (1), and
an imaging unit (58) adapted to obtain an image of an inside of the subject (1) illuminated by the illuminating unit (56).

**10.** The intra-subject position detection system according to claim 9, wherein
the position detecting device (3) further includes a storing unit (74) adapted to store the image obtained by the imaging unit (58) and a position of the subject insertable device (55) at a time the image is obtained in association with each other.

**Patentansprüche**

**1.** System zum Erfassen einer Position in einem Patienten, mit:

einer in einen Patienten (1) einführbaren Vorrichtung (2; 55), die dazu ausgebildet ist, in einen Patienten (1) eingeführt zu werden und sich durch den Patienten (1) hindurch zu bewegen; und
eine Positionserfassungsvorrichtung (3), die dazu ausgebildet ist, außerhalb des Patienten (1) angeordnet zu werden und Positionsinformation der in den Patienten (1) einführbaren und sich im Patienten (1) befindenden Vorrichtung (2; 55) zu ermitteln, wobei
die in den Patienten (1) einführbaren Vorrichtung (2; 55) einen Magnetfeldgenerator (12), der ein magnetostatisches Feld zu erzeugen vermag, aufweist, wobei
die Positionserfassungsvorrichtung (3) enthält:

eine Mehrzahl von Magnetfelddetektoreinrichtungen (6a bis 6h; 36a bis 36h), die dazu ausgebildet sind, zu einem Verwendungszeitpunkt an dem Patienten (1) angebracht zu werden und die jeweilige Stärke des vom Magnetfeldgenerator (12) abgegebenen magnetostatischen Felds zu erfassen,
ein Bezugssensor (7; 37; 41; 50), der dazu ausgebildet ist, Positionen der Magnetfelddetektoreinrichtungen (6a bis 6h; 36a bis 36h) relativ zu dem Bezugssensor (7; 37; 41; 50) abzuleiten, und
eine Positionsinformationsableitungseinheit (10; 70), die dazu ausgebildet ist, eine Position der in den Patienten (1) einführbaren Vorrichtung (2; 55) im Inneren des Patienten (1) abzuleiten, basierend auf den durch die Magnetfelddetektoreinrichtungen (6a bis 6h; 36a bis 36h) erfassten Magnetfeldstärken und den durch den Bezugssensor (7; 37; 41; 50) erfassen Positionen der Magnetfelddetektoreinrichtungen (6a bis 6h; 36a bis 36h), wobei
die Positionserfassungsvorrichtung (3) eine Mehrzahl von ersten Funkeinheiten (16) aufweist, die jeweils an den Magnetfelddetektoreinrichtungen (6a bis 6h; 36a bis 36h) befestigt sind, wobei
der Bezugssensor (7; 37; 41; 50) enthält:

eine zweite Funkeinheit (19; 25, 42 bis 45; 52), die dazu ausgebildet ist, von den ersten Funkeinheiten (16) ausgesendete Funksignale zu empfangen; und
eine Abstandsableitungseinheit (27), die dazu ausgebildet ist, Abstände zwischen dem Bezugssensor (7; 37; 41; 50) und den Positionen der Magnetfelddetektoreinrichtungen (6a bis 6h; 36a bis 36h) abzuleiten, basierend auf an der zweiten Funkeinheit (19; 25, 42 bis 45; 52) empfangenen Signalstärken der Funksignale, und wobei
die Positionsinformationsableitungseinheit (10; 70) dazu ausgebildet ist, auf die Positionen der Magnetfelddetektoreinrichtungen (6a bis 6h; 36a bis 36h) bezogene Information zu erfassen und zu speichern, sodann die Stärken des magnetostatischen Felds durch die Magnetfelddetektoreinrichtungen (6a bis 6h; 36a bis 36h) zu erfassen, Abstände zwischen der in den Patienten (1) einführbaren Vorrichtung (2; 55) und den Magnetfelddetektoreinrichtungen (6a bis 6h; 36a bis 36h) basierend auf den erfassten Stärken abzuleiten, und die Position der in den Patienten (1) einführbaren Vorrichtung (2; 55) basierend auf den Abständen und der Positionsinformation der Magnetfelddetektoreinrichtungen (6a bis 6h; 36a bis 36h) abzuleiten.

**2.** System zum Erfassen einer Position in einem Patienten nach Anspruch 1, wobei
der Bezugssensor (7; 37; 41; 50) dazu ausgebildet ist, die Abstände zwischen dem Bezugssensor (7; 37; 41; 50) und den Positionen der Magnetfelddetektoreinrichtungen (6a bis 6h; 36a bis 36h) abzuleiten und die Positionen der

**EP 1 731 093 B1**

Magnetfelddetektoreinrichtungen (6a bis 6h; 36a bis 36h) relativ zu dem Bezugssensor (7; 37; 41; 50) basierend auf den abgeleiteten Abständen abzuleiten.

3. System zum Erfassen einer Position in einem Patienten nach Anspruch 1, wobei der Bezugssensor (7; 37; 41; 50) ferner enthält:

eine Positionsinformationsdatenbank (22), die dazu ausgebildet ist, eine Übereinstimmung zwischen den Positionen der Magnetfelddetektoreinrichtungen (6a bis 6h) und den Abständen zwischen dem Bezugssensor (7; 37; 41; 50) und jedem der Magnetfelddetektoreinrichtungen (6a bis 6h) zu speichern, und
eine Positionsableitungseinheit (28), die dazu ausgebildet ist, Positionen, die den von der Abstandsableitungseinheit (27) abgeleiteten Abständen zugeordnet sind, aus der in der Positionsinformationsdatenbank (22) abgespeicherten Übereinstimmung zu extrahieren.

4. System zum Erfassen einer Position in einem Patienten nach Anspruch 1, wobei
die zweite Funkeinheit eine Mehrzahl von Empfangsantennen (42, 43, 44, 45) aufweist, die jeweils einer Mehrzahl von Bezugspositionen entsprechen, wobei die Empfangsantennen (42, 43, 44, 45) dazu ausgebildet sind, die von den ersten Funkeinheiten (16) ausgesendete Funksignale zu empfangen,
der Bezugssensor (41) ferner aufweist:

einen Selektor (45), der dazu ausgebildet ist, die von den Empfangsantennen (42, 43, 44, 45) empfangenen Funksignale nacheinander auszugeben,
eine Empfangsschaltung (25), die dazu ausgebildet ist, Stärken eines jeden der nacheinander durch den Selektor (45) ausgegebenen Funksignale zu extrahieren, und
die Abstandsableitungseinheit (27) dazu ausgebildet ist, Abstände zwischen jeder der Bezugspositionen und einer der Positionen der Magnetfelddetektoreinrichtungen (6a bis 6h) für jede der Magnetfelddetektoreinrichtungen (6a bis 6h) abzuleiten, basierend auf den von der Empfangsschaltung (25) extrahierten Stärken, um die Positionen der Magnetfelddetektoreinrichtungen (6a bis 6h) abzuleiten.

5. System zum Erfassen einer Position in einem Patienten nach Anspruch 1, wobei der Bezugssensor (50) ferner enthält:

eine Gruppenantenne (51), die dazu ausgebildet ist, eine Richtung der Magnetfelddetektoreinrichtungen (6a bis 6h; 36a bis 36h) zu erfassen,
eine Orientierungseinstelleinheit (53), die dazu ausgebildet ist, eine Orientierungsrichtung der Gruppenantenne (51) einzustellen, wobei die Orientierungsrichtung eine Richtung ist, in der die Gruppenantenne (51) mit der Richtung der Magnetfelddetektoreinrichtungen (6a bis 6h; 36a bis 36h) übereinstimmt, und
der Bezugssensor (50) dazu ausgebildet ist, die Positionen der Magnetfelddetektoreinrichtungen (6a bis 6h) abzuleiten, basierend auf den von der Abstandsableitungseinheit (27) abgeleiteten Abständen und der von der Orientierungseinstelleinheit (53) eingestellten Orientierungsrichtung.

6. System zum Erfassen einer Position in einem Patienten nach Anspruch 1, wobei
die zweite Funkeinheit (19) dazu ausgebildet ist, das Funksignal an jede der ersten Funkeinheiten (16) in einem Zeitmultiplexmodus zu senden.

7. System zum Erfassen einer Position in einem Patienten nach Anspruch 1, wobei
die zweite Funkeinheit (19) dazu ausgebildet ist, das Funksignal einer verschiedenen Frequenz an jede der ersten Funkeinheiten (16) zu senden.

8. System zum Erfassen einer Position in einem Patienten nach Anspruch 1, wobei die in den Patienten (1) einführbare Vorrichtung (55) ferner enthält:

eine Erlangungseinheit (60) zum Erlangen von im Inneren des Patienten (1) vorgegebener Information, die dazu ausgebildet ist, im Inneren des Patienten (1) Information als das Innere des Patienten (1) betreffende Information zu erlangen, wenn die in den Patienten (1) einführbaren Vorrichtung (2; 55) in den Patienten (1) eingeführt ist und sich durch den Patienten (1) hindurch bewegt, und
eine Funkübertragungseinheit (61, 62), die dazu ausgebildet ist, die das Innere des Patienten (1) betreffende Information, die durch die Erlangungseinheit (60) zum Erlangen von das Innere des Patienten (1) betreffender Information erlangt wurde, durch Funk zu übertragen, und wobei

28

die Positionsnachweisvorrichtung (3) ferner aufweist:

eine Empfangseinheit (A1 bis An, 71, 72), die dazu ausgebildet ist, ein die das Innere des Patienten (1) betreffende Information enthaltendes Funksignal zu empfangen, wobei das Funksignal von der Funkübertragungseinheit (61, 62) gesendet wird.

9. System zum Erfassen einer Position in einem Patienten nach Anspruch 8, wobei die Erlangungseinheit (60) zum Erlangen von das Innere des Patienten (1) betreffender Information enthält:

eine Beleuchtungseinheit (56), die dazu ausgebildet ist, das Innere des Patienten (1) zu beleuchten, und eine Abbildungseinheit (58), die ein Bild des durch die Beleuchtungseinheit (56) beleuchteten Inneren des Patienten (1) abzubilden vermag.

10. System zum Erfassen einer Position in einem Patienten nach Anspruch 9, wobei die Positionserfassungsvorrichtung (3) ferner eine Speichereinheit (74) aufweist, die das durch die Abbildungseinheit (58) abgebildete Bild und eine Position der in den Patienten (1) einführbaren Vorrichtung (55) in Zuordnung zueinander zu einem Zeitpunkt, zu dem das Bild abgebildet wird, zu speichern vermag.

**Revendications**

1. Système de détection de position intra-sujet, comprenant:

un dispositif pouvant être inséré dans un sujet (2; 55) adapté pour être introduit dans un sujet (1) et déplacé à travers le sujet (1); et
un dispositif de détection de position (3) adapté pour être disposé à l'extérieur du sujet (1) et obtenir des informations de position du dispositif pouvant être inséré dans un sujet (2; 55) à l'intérieur du sujet (1), dans lequel le dispositif pouvant être inséré dans un sujet (2; 55) comprend un générateur de champ magnétique (12) adapté pour générer un champ magnétostatique, dans lequel
le dispositif de détection de position (3) comprend
une pluralité d'unités de détection de champ magnétique (6a à 6h; 36a à 36h) adaptées pour être disposées sur le sujet (1) à un moment d'utilisation et détecter les intensités du champ magnétostatique délivré en sortie depuis le générateur de champ magnétique (12),
un capteur de référence (7; 37; 41; 50) adapté pour dériver les positions des unités de détection de champ magnétique (6a à 6h; 36a à 36h) par rapport au capteur de référence (7; 37; 41; 50), et
une unité de dérivation d'informations de position (10; 70) adaptée pour dériver une position du dispositif pouvant être inséré dans un sujet (2; 55) à l'intérieur du sujet (1) sur la base des intensités de champ magnétique détectées par les unités de détection de champ magnétique (6a à 6h; 36a à 36h) et les positions des unités de détection de champ magnétique (6a à 6h; 36a à 36h) détectées par le capteur de référence (7; 37; 41; 50), dans lequel
le dispositif de détection de position comprend une pluralité de premières unités radio (16) fixées aux unités de détection de champ magnétique (6a à 6h; 36a à 36h), respectivement, dans lequel
le capteur de référence (7; 37; 41; 50) comprend:

une deuxième unité radio (19; 25, 42 à 45; 52) adaptée pour recevoir les signaux radio transmis depuis les premières unités radio (16); et
une unité de dérivation de distance (27) adaptée pour dériver les distances entre le capteur de référence (7; 37; 41; 50) et les positions des unités de détection de champ magnétique (6a à 6h; 36a à 36h) sur la base des intensités de signaux reçus des signaux radio au niveau de la deuxième unité radio (19; 25, 42 à 45; 52), et dans lequel
l'unité de dérivation d'informations de position (10; 70) est adaptée pour acquérir et mémoriser des informations se rapportant aux positions des unités de détection de champ magnétique (6a à 6h; 36a à 36h), puis détecter les intensités du champ magnétostatique par les unités de détection de champ magnétique (6a à 6h; 36a à 36h), dériver les distances entre le dispositif pouvant être inséré dans un sujet (2; 55) et les unités de détection de champ magnétique (6a à 6h; 36a à 36h) sur la base des intensités détectées, et dériver la position du dispositif pouvant être inséré dans un sujet (2; 55) sur la base des distances et des informations de position des unités de détection de champ magnétique (6a à 6h; 36a à 36h).

**2.** Système de détection de position intra-sujet selon la revendication 1, dans lequel
le capteur de référence (7; 37; 41; 50) est adapté pour dériver les distances entre le capteur de référence (7; 37; 41; 50) et les positions des unités de détection de champ magnétique (6a à 6h; 36a à 36h), et dériver les positions des unités de détection de champ magnétique (6a à 6h; 36a à 36h) par rapport au capteur de référence (7; 37; 41; 50) sur la base des distances dérivées.

**3.** Système de détection de position intra-sujet selon la revendication 1, dans lequel le capteur de référence (7; 37; 41; 50) comprend en outre:

une base de données d'informations de position (22) qui est adaptée pour mémoriser une correspondance entre les positions des unités de détection de champ magnétique (6a à 6h) et les distances entre le capteur de référence (7; 37; 41; 50) et chacune des unités de détection de champ magnétique (6a à 6h), et
une unité de dérivation de position (28) qui est adaptée pour extraire les positions associées aux distances dérivées par l'unité de dérivation de distance (27) à partir de la correspondance mémorisée dans la base de données d'informations de position (22).

**4.** Système de détection de position intra-sujet selon la revendication 1, dans lequel:

la deuxième unité radio comprend une pluralité d'antennes de réception (42, 43, 44, 45) correspondant à une pluralité de positions de référence, respectivement, les antennes de réception (42, 43, 44, 45) étant adaptées pour recevoir les signaux radio transmis depuis les premières unités radio (16),
le capteur de référence (41) comprend en outre:

un sélecteur (45) adapté pour délivrer séquentiellement en sortie le signal radio reçu par l'intermédiaire de l'antenne de réception (42, 43, 44, 45),
un circuit de réception (25) adapté pour extraire les intensités de chacun des signaux radio séquentiellement délivrés en sortie par le sélecteur (45), et
une unité de dérivation de distance (27) est adaptée pour dériver les distances entre chacune des positions de référence et l'une des positions des unités de détection de champ magnétique (6a à 6h) pour chacune des unités de détection de champ magnétique (6a à 6h) sur la base des intensités extraites par le circuit de réception (25), pour dériver les positions des unités de détection de champ magnétique (6a à 6h).

**5.** Système de détection de position intra-sujet selon la revendication 1, dans lequel le capteur de référence (50) comprend en outre
une antenne réseau (51) adaptée pour détecter une direction des unités de détection de champ magnétique (6a à 6h; 36a à 36h),
une unité d'ajustement d'orientation (53) qui est adaptée pour ajuster une direction d'orientation de l'antenne réseau (51), la direction d'orientation étant une direction dans laquelle l'antenne réseau (51) correspond avec la direction des unités de détection de champ magnétique (6a à 6h; 36a à 36h), et
le capteur de référence (50) est adapté pour dériver les positions des unités de détection de champ magnétique (6a à 6h) sur la base des distances dérivées par l'unité de dérivation de distance (27) et de la direction d'orientation ajustée par l'unité d'ajustement d'orientation (53).

**6.** Système de détection de position intra-sujet selon la revendication 1, dans lequel
la deuxième unité radio (19) est adaptée pour transmettre le signal radio vers chacune des premières unités radio (16) de manière multiplexée dans le temps.

**7.** Système de détection de position intra-sujet selon la revendication 1, dans lequel
la deuxième unité radio (19) est adaptée pour transmettre le signal radio d'une fréquence différente vers chacune des premières unités radio (16).

**8.** Système de détection de position intra-sujet selon la revendication 1, dans lequel
le dispositif pouvant être inséré dans un sujet (55) comprend en outre une unité d'obtention d'informations intra-sujet prédéterminées (60) adaptée pour obtenir des informations de l'intérieur du sujet (1) en tant qu'informations intra-sujet lorsque le dispositif pouvant être inséré dans un sujet (55) est introduit dans le sujet (1) et déplacé à travers le sujet (1), et
une unité de transmission radio (61, 62) adaptée pour transmettre les informations intra-sujet obtenues par l'unité d'obtention d'informations intra-sujet (60) par radio, et dans lequel

le dispositif de détection de position (3) comprend en outre
une unité de réception (Al à An, 71, 72) adaptée pour recevoir un signal radio contenant les informations intra-sujet, le signal radio étant envoyé depuis l'unité de transmission radio (61, 62).

9. Système de détection de position intra-sujet selon la revendication 8, dans lequel
l'unité d'obtention d'informations intra-sujet (60) comprend une unité d'éclairage (56) adaptée pour éclairer un intérieur du sujet (1), et une unité d'imagerie (58) adaptée pour obtenir une image de l'intérieur du sujet (1) éclairé par l'unité d'éclairage (56).

10. Système de détection de position intra-sujet selon la revendication 9, dans lequel
le dispositif de détection de position (3) comprend en outre une unité de mémorisation (74) adaptée pour mémoriser l'image obtenue par l'unité d'imagerie (58) et une position du dispositif pouvant être inséré dans un sujet (55) à un moment où l'image est obtenue en association avec la position.

# FIG.1

SUBJECT
1

TEST CAPSULE
(SUBJECT INSERTABLE DEVICE)
2

POSITION
DETECTING
DEVICE
3

6d    6c

6b

FIXING MEMBER
9a

MAGNETIC FIELD DETECTOR
6a

6h    6g

z

y

REFERENCE SENSOR
7

x

6e    9b    6f

10
POSITION INFORMATION
DERIVING UNIT

DISPLAY DEVICE
4

5
PORTABLE RECORDING MEDIUM

EP 1 731 093 B1

# FIG.2

CASING
11

S   N

12
PERMANENT MAGNET
(MAGNETIC FIELD GENERATOR)

13
FILLING MEMBER

# FIG.3

**MAGNETIC FIELD DETECTOR** ⌐6

**RADIO TRANSMISSION UNIT** ⌐16

18

17
**TRANSMITTER**

15
**MAGNETIC FIELD SENSOR**

**REFERENCE SENSOR** ⌐7

**RADIO RECEPTION UNIT**

19

24

23
**OUTPUT UNIT**

25
**RECEIVING CIRCUIT**

**CONTROL UNIT** — 20

**RECEIVED SIGNAL STRENGTH DETECTING UNIT** — 26

21
**DISTANCE STORAGE UNIT**

**DISTANCE DERIVING UNIT** — 27

22
**CORRESPONDENCE DB**

**POSITION DERIVING UNIT** — 28

34

# FIG.4

POSITION INFORMATION DERIVING UNIT — 10

STRENGTH COMPARATOR — 30

STORAGE UNIT — 35

DISTANCE DERIVING UNIT — 32

SELECTOR — 31

CAPSULE POSITION CALCULATOR — 34

POSITION INFORMATION STORING UNIT — 33

MAGNETIC FIELD DETECTOR — 6a

MAGNETIC FIELD DETECTOR — 6b

MAGNETIC FIELD DETECTOR — 6c

MAGNETIC FIELD DETECTOR — 6d

MAGNETIC FIELD DETECTOR — 6h

REFERENCE SENSOR — 7

EP 1 731 093 B1

# FIG.5

```
                    START

                       │
                       ▼
        ┌─────────────────────────────┐
        │  SELECT MAGNETIC FIELD       │─ S101
        │  DETECTOR                    │
        └─────────────────────────────┘
                       │
                       ▼
        ┌─────────────────────────────┐
        │  RECEIVE RADIO SIGNAL SENT   │─ S102
        │  FROM SELECTED MAGNETIC      │
        │  FIELD DETECTOR              │
        └─────────────────────────────┘
                       │
                       ▼
        ┌─────────────────────────────┐
        │  DETECT RECEIVED SIGNAL      │─ S103
        │  STRENGTH OF RADIO SIGNAL    │
        └─────────────────────────────┘
                       │
                       ▼
        ┌─────────────────────────────┐
        │  DERIVE DISTANCE BETWEEN     │─ S104
        │  MAGNETIC FIELD DETECTOR     │
        │  AND REFERENCE POINT         │
        └─────────────────────────────┘
                       │
                       ▼
        ┌─────────────────────────────┐
        │  STORE RESULT OF DERIVATION  │─ S105
        └─────────────────────────────┘
                       │
                       ▼
                    S106
                   ╱      ╲
                  ╱  HAS    ╲
            ╱ DISTANCE BEEN DERIVED ╲    NO
            ╲ FOR ALL MAGNETIC FIELD ╱─────
                ╲  DETECTORS?  ╱
                   ╲        ╱
                       │ YES
                       ▼
        ┌─────────────────────────────┐
        │  DERIVE POSITION OF MAGNETIC │─ S107
        │  FIELD DETECTOR RELATIVE TO  │
        │  REFERENCE POINT             │
        └─────────────────────────────┘
                       │
                       ▼
        ┌─────────────────────────────┐
        │  OUTPUT POSITION INFORMATION │─ S108
        │  OF MAGNETIC FIELD DETECTOR  │
        └─────────────────────────────┘
                       │
                       ▼
                    END
```

# FIG.6

```
┌─────────────────────┐
│        START        │
└─────────────────────┘
            │
            ▼
┌──────────────────────────────────────────────────────┐
│ STORE POSITION INFORMAITON OF MAGNETIC FIELD DETECTOR │─S201
└──────────────────────────────────────────────────────┘
            │
            ▼
┌──────────────────────────────────────────────────────┐
│           DETECT MAGNETIC FIELD STRENGTH             │─S202
└──────────────────────────────────────────────────────┘
            │
            ▼
┌──────────────────────────────────────────────────────┐
│            SELECT MAGNETIC FIELD DETECTOR            │─S203
└──────────────────────────────────────────────────────┘
            │
            ▼
┌──────────────────────────────────────────────────────┐
│    DERIVE DISTANCE BETWEEN SELECTED MAGNETIC FIELD   │─S204
│           DETECTOR AND TEST CAPSULE                  │
└──────────────────────────────────────────────────────┘
            │
            ▼
┌──────────────────────────────────────────────────────┐
│   DERIVE POSITION OF TEST CAPSULE BASED ON DERIVED   │─S205
│  DISTANCE AND POSITION OF MAGNETIC FIELD DETECTOR    │
└──────────────────────────────────────────────────────┘
            │
            ▼
┌──────────────────────────────────────────────────────┐
│      STORE DERIVED POSITION IN RECORDING MEDIUM      │─S206
└──────────────────────────────────────────────────────┘
            │
            ▼
┌─────────────────────┐
│         END         │
└─────────────────────┘
```

# FIG.7

# FIG.8

REFERENCE SENSOR — 37

RADIO RECEPTION UNIT — 19

24

25
RECEIVING CIRCUIT

21
DISTANCE STORAGE UNIT

22
CORRESPONDENCE DB

CONTROL UNIT — 38

SPECTRUM ANALYZER — 39

DISTANCE DERIVING UNIT — 27

POSITION DERIVING UNIT — 28

OUTPUT UNIT — 23

# FIG.9

MAGNETIC FIELD DETECTOR

RADIO TRANSMISSION UNIT

16

18

17
TRANSMITTER

15
MAGNETIC FIELD SENSOR

6

$r_a$

$r_b$

$r_c$

41
REFERENCE SENSOR

42

43

44

SELECTOR — 45

RECEIVING CIRCUIT — 25

CONTROL UNIT — 46

RECEIVED SIGNAL STRENGTH DETECTING UNIT — 26

DISTANCE DERIVING UNIT — 27

POSITION DERIVING UNIT — 47

21
DISTANCE STORAGE UNIT

23
OUTPUT UNIT

# FIG.10

50

REFERENCE SENSOR

RADIO RECEPTION UNIT

52

ARRAY ANTENNA — 51

RECEIVING CIRCUIT — 25

CONTROL UNIT — 54

RECEIVED SIGNAL STRENGTH DETECTING UNIT — 26

DISTANCE DERIVING UNIT — 27

POSITION DERIVING UNIT — 28

ORIENTATION ADJUSTER — 53

23

OUTPUT UNIT

# FIG.11

CAPSULE ENDOSCOPE — 55

FUNCTION EXECUTING UNIT — 60

| 56 | 57 |
| LED (ILLUMI-NATING UNIT) | LED DRIVING UNIT |

TRANSMITTING ANTENNA UNIT — 62

58 — CCD (IMAGING UNIT)

61 — TRANSMITTING CIRCUIT

59 — CCD DRIVING UNIT

PERMANENT MAGNET — 12

| N | S |

63 — SYSTEM CONTROL CIRCUIT

69 — CONTROL INFORMATION DETECTING CIRCUIT

64 — RECEIVING ANTENNA UNIT

65 — SEPARATING CIRCUIT

66 — POWER REGENERATING CIRCUIT

67 — BOOSTER CIRCUIT

68 — CAPACITOR

FIG.12

POSITION INFORMATION DERIVING UNIT

70

RECEIVING ANTENNA A1
RECEIVING ANTENNA A2
...
RECEIVING ANTENNA An
POWER SUPPLY ANTENNA B1
...
POWER SUPPLY ANTENNA Bm
MAGNETIC FIELD DETECTOR 6a
6b
...
6h
REFERENCE SENSOR 7

71 ANTENNA SELECTOR
72 RECEIVING CIRCUIT
73 SIGNAL PROCESSING CIRCUIT
74 STORAGE UNIT
76 CONTROL INFORMATION INPUT UNIT
75 OSCILLATOR
77 SUPERPOSING CIRCUIT
79 POWER SUPPLY UNIT
78 AMPLIFIER
30 STRENGTH COMPARATOR
31 SELECTOR
32 DISTANCE DERIVING UNIT
34 CAPSULE POSITION CALCULATOR
33 POSITION INFORMATION STORING UNIT

43

# FIG.13

SUBJECT
1

TEST CAPSULE
(SUBJECT INSERTABLE DEVICE)
2

6d    6c

6b

POSITION
DETECTING
DEVICE
103

FIXING MEMBER
9a

MAGNETIC FIELD DETECTING UNIT
6a

6h    6g

POSITION INFORMATION DERIVING UNIT
108

6e    9b  6f

109

ALTERNATE CURRENT MAGNETIC FIELD
GENERATOR

DISPLAY DEVICE
4

5
POTABLE RECORDING MEDIUM

EP 1 731 093 B1

# FIG.14

POSITION INFORMATION DERIVING UNIT 108

ALTERNATING-CURRENT MAGNETIC FIELD GENERATOR 109

DEVICE COORDINATE DERIVING UNIT 119

SUBTRACTER 118

DC COMPONENT REMOVING UNIT (ALTERNATE CURRENT MAGNETIC FIELD EXTRACTING UNIT) 117

MAGNETIC FIELD DETECTOR 6a

STRENGTH COMPARATOR 114

LPF (DIRECT-CURRENT MAGNETIC FIELD EXTRACTING UNIT) 113

STORAGE UNIT 121

POSITION CALCULATOR 120

DISTANCE DERIVING UNIT 116

SELECTOR 115

MAGNETIC FIELD DETECTOR 6h

EP 1 731 093 B1

# FIG.15

START

DERIVE POSITION COORDINATE OF MAGNETIC FIELD DETECTOR — S301

SELECT MAGNETIC FIELD DETECTOR HAVING HIGH MAGNETIC FIELD STRENGTH OF DIRECT-CURRENT MAGNETIC FIELD COMPONENT — S302

ACQUIRE SPECIFIC VALUE OF STRENGTH OF DIRECT-CURRENT MAGNETIC FIELD COMPONENT — S303

DERIVE DISTANCE BETWEEN MAGNETIC FIELD DETECTOR AND TEST CAPSULE — S304

DERIVE POSITION OF TEST CAPSULE — S305

STORE POSITION OF TEST CAPSULE — S306

END

# FIG.16

TEST CAPSULE

# FIG.17

SUBJECT
~1

122
CAPSULE ENDOSCOPE
(SUBJECT INSERTABLE DEVICE)

A4          A5
124d        124c
124b

POSITION
DETECTING
UNIT
123

FIXING MEMBER
9a

MAGNETIC FIELD
DETECTOR
124a

A2    A3
124h    124g

RECEIVING ANTENNA
A1

A6    An

POSITION INFORMATION
DERIVING UNIT
125

124e    9b    124f

109
ALTERNATING-CURRENT MAGNETIC FIELD GENERATOR

# FIG.18

# FIG.19

RECEIVING ANTENNA A1

RECEIVING ANTENNA A2

RECEIVING ANTENNA An

POWER SUPPLY ANTENNA B1

POWER SUPPLY ANTENNA B1

ALTERNATE CURRENT MAGNETIC FIELD GENERATOR 109

MAGNETIC FIELD DETECTOR 124a

124h

POSITION INFORMATION DERIVING UNIT 125

ANTENNA SELECTOR (SELECTING UNIT) 149

RECEIVING CIRCUIT 150

SIGNAL PROCESSING UNIT 151

STORAGE UNIT 152

POWER SUPPLY UNIT 157

CONTROL INFORMATION INPUT UNIT 154

AMPLIFIER 156

SUPERPOSING CIRCUIT 155

OSCILLATOR 153

DC COMPONENT REMOVING UNIT 146

SUBTRACTER 147

DEVICE COORDINATE DERIVING UNIT 148

STRENGTH COMPARATOR 140

ORIENTATION DETECTING UNIT 145

ORIENTATION DB 144

L P F 158

SELECTOR 141

DISTANCE DERIVING UNIT 142

POSITION CALCULATOR 143

EP 1 731 093 B1

# FIG.20

START

INPUT POSITION OF CAPSULE ENDOSCOPE & MAGNETIC FIELD DIRECTION RECEIVED BY SELECTED MAGNETIC FIELD DETECTOR — S401

DERIVE RELATIVE POSITION OF SELECTED MAGNETIC FIELD DETECTOR RELATIVE TO CAPSULE ENDOSCOPE — S402

INPUT RELATIVE POSITION AND MAGNETIC FIELD DIRECTION TO ORIENTATION DATABASE & DERIVE ORIENTATION — S403

OUTPUT ORIENTATION TO ANTENNA SELECTING UNIT AND STORAGE UNIT — S404

END

# FIG.21

MAGNETIC FIELD DETECTOR
124

$(a_1, a_2, a_3)$

$(x_1, y_1, z_1)$

CAPSULE ENDOSCOPE
122

$(x, y, z)$

**EP 1 731 093 B1**

**REFERENCES CITED IN THE DESCRIPTION**

**Patent documents cited in the description**

- JP 2003019111 A
- JP 2004041709 A